(19)

**Europäisches Patentamt**
**European Patent Office**
**Office européen des brevets**

(11) **EP 3 714 909 A1**

(12) **EUROPEAN PATENT APPLICATION**

(43) Date of publication:
**30.09.2020 Bulletin 2020/40**

(21) Application number: **19165892.1**

(22) Date of filing: **28.03.2019**

(51) Int Cl.:
*A61L 27/10* (2006.01)   *A61L 27/12* (2006.01)
*A61L 27/34* (2006.01)   *A61L 27/36* (2006.01)
*A61L 27/42* (2006.01)   *A61L 27/54* (2006.01)
*A61L 27/58* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Industrie Biomediche Insubri S.A.**
**6805 Mezzovico-Vira (CH)**

(72) Inventors:
• **PERALE, Giuseppe**
  **6925 Gentilino in Collina d'Oro (CH)**
• **BETGE, Felice**
  **6946 Ponte Capriasca (CH)**
• **HAUGEN, Håvard Jostein**
  **0376 Oslo (NO)**
• **LYNGSTADAAS, Ståle Petter**
  **1450 Nesoddtangen (NO)**

(74) Representative: **Gregorj S.r.l.**
**Via L. Muratori, 13/b**
**20135 Milano (IT)**

(54) **IMPROVED BONE IMPLANT MATRIX COMPRISING PROLINE-RICH PEPTIDE AND METHOD OF PREPARING THE SAME**

(57)    The present invention deals with a bone implant matrix comprising a base matrix selected from the group comprising:
- acellularized or acellularized non-demineralised bone matrix of any source,
- matrix of natural mineral sources,
- synthetic bioceramics matrix,
or combinations of the above, wherein the surface of said base matrix is coated with an statistically homogeneous composition which is a reinforcing mixture containing at least a bio-degradable polyester or co-polymer thereof, at least a gelatine or hydrolysed gelatine and at least an artificial Proline-Rich Peptide.

EP 3 714 909 A1

SEQUENCE LISTING

<110>   INDUSTRIE BIOMEDICHE INSUBRI SA

<120>   "IMPROVED BONE IMPLANT MATRIX COMPRISING PROLINE-RICH PEPTIDE AND
        METHOD OF PREPARING THE SAME"

<130>   P65760EP00

<160>   9

<170>   PatentIn version 3.5

<210>   1
<211>   92
<212>   PRT
<213>   artificial sequence

<220>
<223>   proline rich peptide


<220>
<221>   seqidno1
<222>   (1)..(92)
<223>   I (Ile) can be an aa independently selected form the group consisting
of: Ala,
        Ile, Leu, Met, Phe, Trp and Val, preferably Ile, Leu, Val and
        Met; S (Ser) can be an aa independently selected from the group
consisting of:
        Asn, Cys, Gln, Ser, Thr and Tyr, preferably Ser and Gln

<400>   1

Pro Ile Ile Pro Ser Ser Ser Pro Ile Ile Pro Ser Ser Pro Ile Ile
1               5                   10                  15


Pro Ile Pro Ser Ser Ser Ser Ser Ser Pro Ser Ser Ser Ser Ser Ser
            20                  25                  30


Pro Ile Ile Pro Ile Pro Ser Ser Ser Pro Ser Ser Pro Ser Pro Ile
        35                  40                  45


Ile Pro Ser Pro Ser Ser Pro Ile Ile Pro Ser Ser Pro Ile Ile Pro
    50                  55                  60


Ser Ser Pro Ile Ile Pro Ser Pro Pro Ile Pro Pro Ile Ile Ile Ile
65                  70                  75                  80


Ile Ile Ile Ile Pro Ile Ile Pro Ile Ile Ile Ile
                85                  90


<210>   2
<211>   25
<212>   PRT
<213>   artificial sequence

```
<220>
<223>  proline rich peptide


<220>
<221>  PEPTIDE
<222>  (1)..(25)
<223>  I (Ile) can be an aa independently selected form the group consisting
of: Ala,
        Ile, Leu, Met, Phe, Trp and Val; S (Ser) can be an aa independently
selected
        from the group consisting of: Asn, Cys, Gln, Ser, Thr and Tyr


<400>  2

Pro Ile Ile Pro Ser Ser Pro Ile Ile Pro Ser Ser Pro Ile Ile Pro
1               5                   10                  15


Ser Ser Pro Ser Pro Pro Ile Pro Pro
            20                  25



<210>  3
<211>  25
<212>  PRT
<213>  Artificial Sequence

<220>
<223>  proline rich peptide


<220>
<221>  PEPTIDE
<222>  (1)..(25)

<400>  3

Pro Leu Val Pro Ser Tyr Pro Leu Val Pro Ser Tyr Pro Leu Val Pro
1               5                   10                  15


Ser Tyr Pro Tyr Pro Pro Leu Pro Pro
            20                  25



<210>  4
<211>  25
<212>  PRT
<213>  Artificial Sequence

<220>
<223>  proline rich peptide


<220>
<221>  PEPTIDE
<222>  (1)..(25)

<400>  4

Pro Leu Val Pro Ser Gln Pro Leu Val Pro Ser Gln Pro Leu Val Pro
1               5                   10                  15
```

Ser Gln Pro Gln Pro Pro Leu Pro Pro
                20                  25


<210>   5
<211>   25
<212>   PRT
<213>   Artificial Sequence

<220>
<223>   proline rich peptide


<220>
<221>   PEPTIDE
<222>   (1)..(25)

<400>   5

Pro Leu Val Pro Cys Cys Pro Leu Val Pro Cys Cys Pro Leu Val Pro
1                   5                   10                  15


Cys Cys Pro Cys Pro Pro Leu Pro Pro
                20                  25


<210>   6
<211>   25
<212>   PRT
<213>   Artificial Sequence

<220>
<223>   proline rich peptide


<220>
<221>   PEPTIDE
<222>   (1)..(25)

<400>   6

Pro Met Met Pro Ser Tyr Pro Met Met Pro Ser Tyr Pro Met Met Pro
1                   5                   10                  15


Ser Tyr Pro Tyr Pro Pro Met Pro Pro
                20                  25


<210>   7
<211>   25
<212>   PRT
<213>   Artificial Sequence

<220>
<223>   proline rich peptide


<220>
<221>   PEPTIDE

```
<222>  (1)..(25)

<400>  7

Pro Leu Val Pro Ser Ser Pro Leu Val Pro Ser Ser Pro Leu Val Pro
1               5               10              15


Ser Ser Pro Ser Pro Pro Leu Pro Pro
            20          25


<210>  8
<211>  25
<212>  PRT
<213>  Artificial Sequence

<220>
<223>  proline rich peptide


<220>
<221>  PEPTIDE
<222>  (1)..(25)

<400>  8

Pro Leu Val Pro Ser Ser Pro Leu Val Pro Cys Cys Pro Leu Val Pro
1               5               10              15


Cys Cys Pro Ser Pro Pro Leu Pro Pro
            20          25


<210>  9
<211>  25
<212>  PRT
<213>  Artificial Sequence

<220>
<223>  proline rich peptide


<220>
<221>  PEPTIDE
<222>  (1)..(25)

<400>  9

Pro His Gln Pro Met Gln Pro Gln Pro Pro Val His Pro Met Gln Pro
1               5               10              15


Leu Pro Pro Gln Pro Pro Leu Pro Pro
            20          25
```

## Description

## Technical field of the invention

The present invention relates to implant matrixes to be used generally in at least one of the following fields: bone reconstructive surgery, bone regeneration surgery, regenerative surgery, skull and maxillo-facial bone reconstructive surgery, oncologic surgery, paediatric cases surgery, oral surgery, dental surgery, orthopaedic surgery, spine surgery, traumatology and implantology, and all of these for the paediatric cases, such as bone reconstructive surgery, bone regeneration surgery, regenerative surgery, skull and maxillo-facial bone reconstructive surgery, oncologic surgery, oral surgery, dental surgery, orthopaedic surgery, spine surgery, traumatology and implantology.

State of the art

A frequently used method to treat bone defects is the use of bone grafts to promote tissue regeneration. This as it provides an osteoconductive, osteoinductive and/or osteogenic environment that promotes bone repair and healing [Y. Fillingham, J. Jacobs, Bone grafts and their substitutes, The bone & joint journal 98(1_Supple_A) (2016) 6-9]. Currently autografting, the transplantation of tissue from one site of the patient's body to another, represent the gold standard [L. Roseti, V. Parisi, M. Petretta, C. Cavallo, G. Desando, I. Bartolotti, B. Grigolo, Scaffolds for Bone Tissue Engineering: State of the art and new perspectives, Mater Sci Eng C 78 (2017) 1246-1262]. However, this method has drawbacks such as limited supply and the risk of donor site morbidity [R. Agarwal, A.J. Garcia, Biomaterial strategies for engineering implants for enhanced osseointegration and bone repair, Adv Drug Deliver Rev 94 (2015) 53-62.]. Moreover, there has been reported that 31% of patients experiences persistent pain at the donor site 2 years after operation [D.L. Skaggs, M.A. Samuelson, J.M. Hale, R.M. Kay, V.T. Tolo, Complications of posterior iliac crest bone grafting in spine surgery in children, Spine 25(18) (2000) 2400-2402]. This has motivated the search for alternative bone graft sources, where particularly synthetic grafts of biopolymers, bioceramics, and their composites have received a lot of research focus lately [B. Huang, G. Caetano, C. Vyas, J.J. Blaker, C. Diver, P. Bártolo, Polymer-Ceramic Composite Scaffolds: The Effect of Hydroxyapatite and -tri-Calcium Phosphate, Materials 11(1) (2018) 129; M. Domingos, A. Gloria, J. Coelho, P. Bartolo, J. Ciurana, Three-dimensional printed bone scaffolds: The role of nano/micro-hydroxyapatite particles on the adhesion and differentiation of human mesenchymal stem cells, Proceedings of the Institution of Mechanical Engineers, Part H: Journal of Engineering in Medicine 231(6) (2017) 555-564]. However the synthetic grafts traditionally tends to lag behind on the biological performance compared to autografts, allografts and xenografts [G. Hannink, J.C. Arts, Bioresorbability, porosity and mechanical strength of bone substitutes: what is optimal for bone regeneration?, Injury 42 (2011) S22-S25; S. Corbella, S. Taschieri, R. Weinstein, M. Del Fabbro, Histomorphometric outcomes after lateral sinus floor elevation procedure: a systematic review of the literature and meta-analysis, Clin Oral Implan Res 27(9) (2016) 1106-1122]. When that's said, a former study comparing sinus floor augmentation with anorganic bovine bone (Bio-Oss®, Geistlich AG, Wolhusen, Switzerland) and biphasic calcium phosphate (Straumann® BoneCeramic, Institute Straumann AG, Basel, Switzerland) showed no statistical difference between the bone formation for the two groups [L. Cordaro, D.D. Bosshardt, P. Palattella, W. Rao, G. Serino, M. Chiapasco, Maxillary sinus grafting with Bio-Oss® or Straumann® Bone Ceramic: histomorphometric results from a randomized controlled multicenter clinical trial, Clin Oral Implan Res 19(8) (2008) 796-803], illustrating that commercial research on synthetic bone grafts are catching up.

Again, looking at the academic research environment, many of the cited studies uses additive manufacturing systems to produce their grafts. Although 3D printing has a promising potential, particularly when it comes to customized implants, the implementation is still limited by high production cost and long production time [A. Aldaadaa, N. Owji, J. Knowles, Three-dimensional Printing in Maxillofacial Surgery: Hype versus Reality, Journal of tissue engineering 9 (2018) 2041731418770909]. The current commercial market for bone grafts primarily consists of devices based on the refinement of bovine mineral matrix. A selective set of examples for treatment of orthopaedic defects are SmartBone® (IBI-SA, Switzerland), Bio-Oss® (Geistlich, Switzerland) and maxgraft® (Biotiss, Germany - Processed human allograft). Treatment of a larger bone block does generally have a higher productivity and lower cost than 3D printing scaffolds.

Even though there are many commercially available bone graft substitutes, there are none particularly addressing the paediatric market. There has been stable reports of that fractures represents 20-30% of all diagnosis for children [P.-E. Fournier, R. Rizzoli, D.O. Slosman, G. Theintz, J.-P. Bonjour, Asynchrony between the rates of standing height gain and bone mass accumulation during puberty, Osteoporosis Int 7(6) (1997) 525-532]. This means that there is an increasing treatment demand with increased population size. The paediatric market is unique as the skeletal immaturity of the patients is linked to considerable further bone growth [N.E. Picardo, G.W. Blunn, A.S. Shekkeris, J. Meswania, W.J. Aston, R.C. Pollock, J.A. Skinner, S.R. Cannon, T.W. Briggs, The medium-term results of the Stanmore non-invasive extendible endoprosthesis in the treatment of paediatric bone tumours, The Journal of Bone and Joint Surgery. British volume 94-B(3) (2012) 425-430.], inducing very high performance requirements to medical devices. During puberty, the human body experiences a very high increase in physical dimensions, however the increase in bone mass seems to lag behind [P.-E. Fournier, R. Rizzoli, D.O. Slosman, G. Theintz, J.P. Bonjour, Asynchrony between the rates of standing height gain and bone mass accumulation during puberty, Osteoporosis Int 7(6) (1997) 525-532]. Thus, the growth mechanism the bone graft should mimic is very complex. This means that there is necessary to develop a device that regulates the bone regeneration in a manner that stimulates biomineralization at a high rate, but only when necessary. Moreover, the long-term effects of bone morphogenic proteins (BMP), the most abundantly used growth factor for bone tissue regeneration, are not clearly identified, which prevents it from being FDA-approved for paediatric treatment [K.M. Emara, R.A. Diab, A.K. Emara, Recent biological trends in management of fracture non-union, World journal of orthopedics 6(8) (2015) 623; S. Boraiah, O. Paul, D. Hawkes, M. Wickham, D.G. Lorich, Complications of recombinant human BMP-2 for treating complex tibial plateau fractures: a preliminary report, Clinical Orthopaedics and Related Research® 467(12) (2009) 3257-3262]. This leads to the demand for a new type of growth factor with improved properties and biocompatibility.

Lately there has been observed that intrinsically disordered proteins (IDP) plays a crucial role in biomineralization, particularly through signalling and regulation of the direction and extend of crystal growth [L. Kalmar, D. Homola, G. Varga, P. Tompa, Structural disorder in proteins brings order to crystal growth in biomineralization, Bone 51(3) (2012) 528-534]. This by affecting chemical and cellular events such as cell signalling, macromolecular self-assembly, protein removal, and crystal nucleation and growth [M. Wojtas, P. Dobryszycki, A. Ozyhar, Intrinsically disordered proteins in biomineralization, Advanced Topics in Biomineralization, InTech2012]. IDPs are recognized by that they are in whole or in part heterogeneously ensembled of flexible molecules, in an unorganized manner, causing its 3D structure to be undefined [M. Wojtas, P. Dobryszycki, A. O y har, Intrinsically disordered proteins in biomineralization, Advanced Topics in Biomineralization, InTech2012; J. Habchi, P. Tompa, S. Longhi, V.N. Uversky, Introducing protein intrinsic disorder, Chemical reviews 114(13) (2014) 6561-6588]. This makes them highly flexible molecules and recent results suggests that IDPs with tuned disorder-order ratio can be used to program the mineralization process, yielding growth of aligned nanocrystals into hierarchical mineralized structures [Elsharkawy, M. Al-Jawad, M.F. Pantano, E. Tejeda-Montes, K. Mehta, H. Jamal, S. Agarwal, K. Shuturminska, A. Rice, N.V. Tarakina, Protein disorder-order interplay to guide the growth of hierarchical mineralized structures, Nature communications 9(1) (2018) 2145]. Some of these peptides has already been used commercially in medical biomineralization. For instance, does Straumann AG (Basel, Switzerland) produce and sell the Straumann® Emdogain® (EMD), a gel-coating primarily based on amelogenin for enamel regeneration. Although some IDPs have been used commercially, the lack of any commercially available bone grafts particularly for the paediatric market denotes that there is the need, in the field of regenerative bone surgery, to find new bone implant matrixes, which have satisfactory characteristics to be used, in particular as a paediatric bone graft, for clinical categories of defects such as Trauma induced skull bone loss, skull bone defects or oncology required bone segment removal (tumour treatment).

Summary of the invention

Object of the present invention is a matrix for bone implant, comprising a base matrix, which is selected from the group comprising:

- acellularized or acellularized non-demineralised bone matrix of any source, i.e. acellularized or acellularized non-demineralised human-derived bone matrix, acellularized or acellularized non-demineralised xeno-derived bone matrix, such as acellularized or acellularized non-demineralised bovine-bone matrix, acellularized or acellularized non-demineralised equine-bone matrix, acellularized or acellularized non-demineralised porcine-bone matrix;
- matrix of natural mineral sources like mother of pearl, coral, nacre;
- synthetic bioceramics matrix such as hydroxyl-apatites, calcium carbonates, calcium phosphates, silicon oxides, titanium oxides, aluminium oxides, zirconia oxides, graphites, bioglasses,

or combinations of the above, the surface of said base matrix is coated with an statistically homogeneous composition which is a reinforcing mixture containing at least a polymer, at least a substance, which is able to promote the cell-rooting and the cell growth, by stimulating cell proliferation and tissue integration ("friendliness to cell") and at least an artificial Proline-Rich Peptide, also defined as PRP (Proline-Rich Peptide) according to the present invention. Preferred embodiments of the bone implant matrix are defined in claims from 2 to 20.

The bone implant matrix object of the present invention is suitable to be used in the bone reconstructive surgery field in general, in orthopaedics, in traumatology, in oncology, in spine surgery and in the oral surgery, in the maxillo-facial and dental implantology, or more in general for use in human or veterinary treatment.

Accordingly, it is a further object of the present invention a bone implant matrix herewith disclosed for use in at least one of the following fields: bone reconstructive surgery, in maxillo-facial bone reconstructive surgery, in oral surgery, dental surgery, orthopaedic surgery and implantology, more in general for use in human or veterinary treatment.

It is a further object of the present invention a method for preparing a bone implant matrix herewith disclosed, said method comprising the steps of:

a) preparing a solution of a reinforcing mixture containing at least a soluble polymer, at least a substance, which is able to promote the cell-rooting and the cell growth, by stimulating cell proliferation and tissue integration ("friendliness to cell") and at least an artificial Proline-Rich Peptide also defined as PRP according to the present invention,
b) immersing a base matrix into said reinforcing mixture solution,
c) drying and optionally degassing the matrix for removing possible solvent residues;
d) optionally immerse the dried and optionally degassed, again, in said solution of reinforcing matrix.

List of figures

Particular embodiments of the invention are described in detail herein below, as a way of example and not limited to, with reference to the attached figures, wherein:

- Figure 1: Mass Release profile in μg of SB with SEQ ID 8+FITC in 2 ml saline water versus time in days;
- Figure 2: Released mass of SB with SEQ ID 8 and 4 with FITC on N and C terminal in μg versus time in days for PRP P2 and P5 in 10 ml purified water. triangle: P2 (SEQ ID 4), circle: P5 (SEQ ID 8). Black filled circle and triangle: FITC attached to C-terminal, non-filled triangle and circle: FITC attached to N-terminal. Fluorescein isothiocyanate (FITC) is a fluorochrome from the derivative of fluorescein. FITC has excitation and emission spectrum peak wavelengths of approximately 495 nm and 519 nm and it is here used as fluorescent label only.
- Figure 3: SEM analysis of random points on the external surface of the SBP. A: SEM photo and C: EDS analysis of bone phase (base matrix). B: SEM photo and D: EDS analysis of polymer phase (reinforcing mixture);
- Figure 4: SEM analysis of random points on the external surface of the SBP. A: SEM photo and C: EDS analysis of bone phase (base matrix). B: SEM photo and D: EDS analysis of polymer phase (reinforcing mixture);
- Figure 5: SEM visualization of MC3T3-E1 cells growing on the surface of samples for 2 and 14 days. Images presented are from a representative superficial area;
- Figure 6: Confocal micrographs of MC3T3-E1 cells cultured for 2 and 14 days on SBP samples. Cells were stained with Phalloidin-FITC (stains actin filaments, elongated fibres) and DAPI (stains nucleus, round structures). The upper row of each day shows depth projection micrographs, images are 2D reconstructions of sections acquired repeatedly in sequential steps along the z-axis. The greyscale code on these rows corresponds to the z-axis depth of DAPI-stained nucleus, coded from dark at 0 μm and light at 60μm depth.
- Figure 7: Cytotoxicity levels for the SBP variants with P2 (SBP2), P5 (SBP5) and P6 (SBP6). The results showed no toxicity towards the pre-osteoblastic cell line. Values represent mean ± SEM. No statistical difference between groups;
- Figure 8: Metabolic activity for SBP2, SBP5 and SBP6 after 2 (2d), 6 (6d) and 14 (14d) days in cell culture; values represent mean ± SEM. No statistical difference between the groups;
- Figure 9: ALP activity for the different groups after 14 days in cell culture. Values represent mean ± SEM;
- Figure 10: RT-PCR analysis of mRNA levels for PRP P2 (-H: high; -M: medium; -L: low, concentration), P5, P6 as SBP2H, SBP2M, SBP2L, SBP5, SBP6 respectively and EMD (Straumann Emdogain®) as a percentage of the control value from day 1 (C). COL1A1: Collagen type 1 Alpha 1 chain; ALP: alkaline phosphate; OC: osteocalcin; VEGFA: vascular endothelial growth factor A. Values represent mean ± SEM. The data shows enhance bone cell responses to the added peptides sequences.
- Figure 11: Cone Beam Computer Tomography (CBCT) image of a pig skull after 8 weeks. The round circles shows the positive and negative control. SBP variants with SEQ ID 4 (SBP2) and SEQ ID 9 (SBP6). The CBCT images show enhanced bone growth around the defects with the peptide sequences 4 and 9 (SBP6:, black box right, SBP2: black box left, empty (negative control): circle right and Smartbone® (positive control): circle
- Figure 12: micro Computer Tomography of a skull defect with SBP variants with SEQ ID 4 (SBP2) showing ingrowth of bone into the defect.

Definitions

In the present context, an "artificial peptide" refers to a peptide that is a non-natural peptide in the sense that it does not normally occur in nature but is the product of amino acids put together and selected in an order, amount and manner generating peptides suitable for use in the context of the present invention. An "artificial peptide" is still a peptide embraced by the present invention even though it might encompass parts of or a whole peptide which happens to be present in nature. "Artificial" may be used interchangeably with terms such as "synthetic" or "non-natural".
In the present context "Pro" denotes the amino acid proline.
In the present context "X" denotes a hydrophobic amino acid. A hydrophobic amino acid is, in the present context, defined as an amino acid selected from the group consisting of: Ala, Ile, Leu, Met, Phe, Trp and Val.

In the present context "Y" denotes a polar amino acid.

A polar ("hydrophilic") amino acid is, in the present context, defined as an amino acid selected from the group consisting of: Asn, Cys, Gln, Ser, Thr and Tyr.

In the present context, common nomenclature is used for denoting amino acids. Therefore, for example, A is Ala (hydrophobic), C is Cys (polar), F is Phe (hydrophobic), H is His, I is Ile (hydrophobic), L is Leu (hydrophobic), M is Met (hydrophobic), N is Asn (polar), Q is Gln (polar), S is Ser (polar), T is Thr (polar), V is Val (hydrophobic), W is Trp (hydrophobic), Y is Tyr (polar)

In the present context "subject" relates to any vertebrate animal, such as bird, reptiles, mammals, primates and humans.

In the present context "PRP" relates to the artificial Proline-Rich Peptide selected from: an artificial peptide comprising the amino acid sequence of Pro-X-X-Pro-Y-Y-Y-Pro-X-X-Pro-Y-Y-Pro-X-X-Pro-X-Pro-Y-Y-Y-Y-Y-Pro-Y-Y-Y-Y-Y-Pro-X-X-Pro-X-Pro-Y-Y-Y-Pro-Y-Y-Pro-Y- Pro-X-X-Pro -Y-Pro- Y-Y-Pro-X-X-Pro-Y-Y-Pro-X-X-Pro-Y-Y -Pro-X-X - Pro-Y-Pro-Pro-X-Pro-Pro -X-X-X-X-X-X-X-Pro-X-X-Pro-X-X-X-X (SEQ ID NO 1), wherein:

a) Pro is proline;

b) X is an amino acid independently selected from the group consisting of Ala, Ile, Leu, Met, Phe, Trp and Val, preferably Ile, Leu, Val and Met;

c) Y, is an amino acid independently selected from the group consisting of Asn, Cys, Gln, Ser, Thr and Tyr, preferably Ser and Gln. or

an artificial peptide comprising the amino acid sequence of Pro-X-X-Pro-Y-Y-Pro-X-X-Pro-Y-Y- Pro-X-X-Pro-Y-Y-Pro-Y-Pro-Pro-X-Pro-Pro (SEQ ID NO 2), wherein:

a) Pro is proline;

b) X is an amino acid independently selected from the group consisting of Ala, Ile, Leu, Met, Phe, Trp and Val;

c) Y is an amino acid independently selected from the group consisting of Asn, Cys, Gln, Ser, Thr and Tyr.

Detailed description of the invention

A bone implant matrix, according to an embodiment of the invention, comprises:

a base matrix the surface thereof is coated with a statistically homogeneous composition which is a reinforcing mixture containing at least a soluble polymer, at least a substance, which is able to promote the cell-rooting and the cell growth, by stimulating cell proliferation and tissue integration ("friendliness to cell") and at least an artificial Proline-Rich Peptide wherein the base matrix is selected from the group comprising:

- acellularized or acellularized non-demineralised bone matrix of any source, i.e. acellularized or acellularized non-demineralised human-derived bone matrix, acellularized or acellularized non-demineralised xeno-derived bone matrix, such as acellularized or acellularized non-demineralised bovine-bone matrix, acellularized or acellularized non-demineralised equine-bone matrix, acellularized or acellularized non-demineralised porcine-bone matrix;
- matrix of natural mineral sources like mother of pearl, coral, nacre;
- synthetic bioceramics matrix such as hydroxyl-apatites, calcium carbonates, calcium phosphates, silicon oxides, titanium oxides, aluminium oxides, zirconia oxides, graphites, bioglasses;

or combinations of the above, the soluble polymer of the reinforcing mixture is a biodegradable polyester or co-polymer thereof, the "friendliness to cell" is selected from the group consisting of gelatine, such as bovine and/or porcine gelatine, hydrolysed gelatine, such as bovine and/or porcine hydrolysed gelatine and the artificial Proline-Rich Peptide belonging to Intrinsic Disorder Protein (IDP), also defined as PRP, according to the present invention, is selected from:

an artificial peptide comprising the amino acid sequence of Pro-X-X-Pro-Y-Y-Y-Pro-X-X-Pro-Y-Y-Pro-X-X-Pro-X-Pro-Y-Y-Y-Y-Y-Pro-Y-Y-Y-Y-Y-Pro-X-X-Pro-X-Pro-Y-Y-Y-Pro-Y-Y-Pro-Y- Pro-X-X-Pro -Y-Pro- Y-Y-Pro-X-X-Pro-Y-Y-Pro-X-X-Pro-Y-Y -Pro-X-X -Pro-Y-Pro-Pro-X-Pro-Pro -X-X-X-X-X-X-X-Pro-X-X-Pro-X-X-X-X (SEQ ID NO 1), wherein:

a) Pro is proline;

b) X is an amino acid independently selected from the group consisting of Ala, Ile, Leu, Met, Phe, Trp and Val, preferably Ile, Leu, Val and Met;

c) Y, is an amino acid independently selected from the group consisting of Asn, Cys, Gln, Ser, Thr and Tyr, preferably Ser and Gln. or

an artificial peptide comprising the amino acid sequence of Pro-X-X-Pro-Y-Y-Pro-X-X-Pro-Y-Y- Pro-X-X-Pro-Y-Y-Pro-Y-Pro-Pro-X-Pro-Pro (SEQ ID NO 2), wherein:

a) Pro is proline;
b) X is an amino acid independently selected from the group consisting of Ala, Ile, Leu, Met, Phe, Trp and Val;
c) Y is an amino acid independently selected from the group consisting of Asn, Cys, Gln, Ser, Thr and Tyr.

Accordingly, the present invention deals with a bone implant matrix comprising a base matrix selected from the group comprising:

- acellularized or acellularized non-demineralised bone matrix of any source, i.e. acellularized or acellularized non-demineralised human-derived bone matrix, acellularized or acellularized non-demineralised xeno-derived bone matrix, such as acellularized or acellularized non-demineralised bovine-bone matrix, acellularized or acellularized non-demineralised equine-bone matrix, acellularized or acellularized non-demineralised porcine-bone matrix;
- matrix of natural mineral sources like mother of pearl, coral, nacre;
- synthetic bioceramics matrix such as hydroxyl-apatites, calcium carbonates, calcium phosphates, silicon oxides, titanium oxides, aluminium oxides, zirconia oxides, graphites, bioglasses;

or combinations of the above; wherein the surface of said base matrix is coated with an statistically homogeneous composition which is a reinforcing mixture containing at least a bio-degradable polyester or co-polymer thereof, at least a gelatine or hydrolysed gelatine and at least an artificial Proline-Rich Peptide, also defined as PRP, according to the present invention, said bone implant matrix identified also as "SBP".

## BASE MATRIX.

By the expression "base matrix" a substantially solid tri-dimensional body is meant, intended, typically porous, after a treatment described herein below, to be implanted in bone cavities.
The base matrix according to the present invention is a base matrix selected from the group comprising:

- acellularized or acellularized non-demineralised bone matrix of any source, i.e. acellularized or acellularized non-demineralised human-derived bone matrix, acellularized or acellularized non-demineralised xeno-derived bone matrix, such as acellularized or acellularized non-demineralised bovine-bone matrix, acellularized or acellularized non-demineralised equine-bone matrix, acellularized or acellularized non-demineralised porcine-bone matrix;
- matrix of natural mineral sources like mother of pearl, coral, nacre;
- synthetic bioceramics matrix such as hydroxyl-apatites, calcium carbonates, calcium phosphates, silicon oxides, titanium oxides, aluminium oxides, zirconia oxides, graphites, bioglasses,,

or combinations of the above.
The base matrix according to the present invention is, in particular, an acellularized or acellularized non-demineralised bone matrix of any source; preferably an acellularized or acellularized non-demineralised human-derived bone matrix or an acellularized or acellularized non-demineralised xeno-derived bone matrix; more preferably an acellularized or acellularized non-demineralised xeno-derived bone matrix selected from the group comprising acellularized or acellularized non-demineralised bovine-bone matrix, acellularized or acellularized non-demineralised equine-bone matrix, acellularized or acellularized non-demineralised porcine-bone matrix; most preferably an acellularized or acellularized non-demineralised bovine-bone matrix.
As it is known, the acellularised bone matrixes are most commonly non-demineralised matrixes completely (or substantially) devoid of the donor's cellular material: i.e. acellularised or acellularised non-demineralised bone matrix of any source.
The bone implant matrixes according to the present invention may have different shapes and dimensions, such as to be adapted according to the shape and the dimensions of the bone cavities, where said matrixes can be implanted. For example, such bone matrixes may be parallelepiped-shaped, in particular cube-shaped.
The dimensions of the bone implant matrixes, for example, can vary from a few mm till some dm of maximum length. Basic matrixes are usually porous.
The base matrix and the polymer(s) of the reinforcing mixture are advantageously bio-compatible.
Furthermore, the base matrix and/or the polymer of the reinforcing mixture are preferably bio-integrable, in order to better assist the growth of the new bone integrated with the surrounding tissue.

**REINFORCING MIXTURE**

Moreover by the expression "reinforcing mixture" it is meant a mixture comprising at least a polymer, i.e. which comprises only one polymer or, alternatively, it may be multi-polymeric, i.e. may comprise more than one polymer at the same time, in combination with at least a substance, which is able to promote the cell-rooting and the cell growth, by stimulating cell proliferation and tissue integration ("friendliness to cell") and at least an artificial Proline-Rich Peptide, as described above.

In particular, by the expression reinforcing mixture it is meant a mixture, wherein the synthetic or natural, and advantageously bio-compatible, polymer or polymers, are finely dispersed.

The reinforcing mixture according to the present invention is obtained starting from two solutions, one of them made of a soluble polymer: a biodegradable polyester or co-polymer thereof, the other one made of a "friendliness to cell": gelatine or hydrolysed gelatine and an artificial Proline-Rich Peptide according to the present invention, added to the same solvent, said two solutions immiscible to each other, but made partially miscible by adding an alcohol or another proper solvent in each of them; in order to obtain a fine and homogeneous molecular dispersion of the at least three components of the reinforcing mixture. Said dispersion during the solvent evaporation step creates a statistically homogeneous coating composition, finely dispersed on the surface of the porous bone matrix, *i.e.* coating it also in the inner cavities, without closing them, though.

**SOLUBLE POLYMER/S**

The solvents used to prepare the reinforcing mixture are among those commonly known in the state of the art to be used according to the identified nature of the components of the reinforcing mixture and may be, for instance, water, dichloromethane, tetrahydrofuran, isopropanol, etc., and their specific use results from the kind of polymer/s, gelatine or hydrolysed gelatine, an artificial Proline-Rich Peptide according to the present invention, used, in view of the well-known laws of chemistry.

The polymer of the reinforcing mixture may be selected, for example, from the group comprising biodegradable polymers, polyesters are preferred, in particular polylactic acid (PLA), polyglycolic acid (PGA), polycaprolactone (PCL) and co-polymers thereof, such as for example polycaprolactone-polylactic (PLA/PCL) co-polymers, poly(L-lactide-co-$\varepsilon$ lactone) co-polymers.

Moreover, the polymer may be selected from the group comprising starch, poly(caprolactones), poly(L-lactide), poly(D,L-lactide-co-glycolide), poly(L-lactide-co-D,L-lactide), their enantiomers, their co-polymers and mixtures thereof. The most preferred polymer/s and or co-polymers are polycaprolactone-polylactic copolymer (PLA/PCL) or poly(L-lactide-co- -caprolactone) co-polymer.

According to the invention, the reinforcing mixture may comprise, beside the polymer or the polymers: such as at least a biodegradable polyester or co-polymer thereof, at least an artificial Proline-Rich Peptide, and at least a "friendliness to cell".

**FRIENDLINESS TO CELL**

By the expression "friendliness to cell" it is meant a substance, which is able to promote the cell-rooting and the cell growth, by stimulating cell proliferation and tissue integration.

According to the present invention the "friendliness to cell" are selected from gelatine, hydrolysed gelatine, in particular porcine or bovine or of any other natural origin gelatine and/or porcine or bovine or of any other natural origin hydrolysed gelatine as "friendliness to cell" is particularly preferred. The presence of at least one "friendliness to cell" assists the cell rooting and growth, since the cell proliferation and the tissue integration are promoted and this is an important advantage in respect to the prior art.

**THE ARTIFICIAL PROLINE-RICH PEPTIDE**

In a preferred embodiment of the present invention, the artificial Proline-Rich Peptide is an artificial peptide comprising the amino acid sequence selected from the group comprising:

PLV PSY PLV PSY PLV PSY PYP PLPP (SEQ ID NO 3),
PLV PSQ PLV PSQ PLV PSQ PQP PLPP (SEQ ID NO 4),
PLV PCC PLV PCC PLV PCC PCP PLPP (SEQ ID NO 5),
PMM PSY PMM PSY PMM PSY PYP PMPP (SEQ ID NO 6),

PLV PSS PLV PSS PLV PSS PSP PLPP (SEQ ID NO 7),
PLV PSS PLV PCC PLV PCC PSP PLPP (SEQ ID NO 8),
PHQ PMQ PQP PVH PMQ PLP PQP PLPP (SEQ ID NO 9)

or a combination thereof,
wherein C is Cys, L is Leu, M is Met, Q is Gln, S is Ser, V is Val, Y is Tyr, H is His, P is Pro; more preferably the artificial Proline-Rich Peptide is an artificial peptide SEQUENCE (N-terminus to C-terminus) selected from the group comprising the amino acid sequence of:

PLV PSQ PLV PSQ PLV PSQ PQP PLPP (SEQ ID NO 4),
PLV PSS PLV PCC PLV PCC PSP PLPP (SEQ ID NO 8),
PHQ PMQ PQP PVH PMQ PLP PQP PLPP (SEQ ID NO 9)

or a combination of the amino acid sequence of:

PLV PSQ PLV PSQ PLV PSQ PQ PPLPP (SEQ ID NO 4) and
PLV PSS PLV PCC PLV PCC PSP PLPP (SEQ ID NO 8),
PLV PSQ PLV PSQ PLV PSQ PQP PLPP (SEQ ID NO 4) and
PHQ PMQ PQP PVH PMQ PLP PQP PLPP (SEQ ID NO 9),
PLV PSS PLV PCC PLV PCC PSP PLPP (SEQ ID NO 8) and
PHQ PMQ PQP PVH PMQ PLP PQP PLPP (SEQ ID NO 9),

wherein C is Cys, L is Leu, M is Met, Q is Gln, S is Ser, V is Val, Y is Tyr, H is His, P is Pro; the most preferred artificial Proline-Rich Peptide is an artificial peptide SEQUENCE (N-terminus to C-terminus) comprising the amino acid sequence of (P6) PHQ PMQ PQP PVH PMQ PLP PQP PLPP (SEQ ID NO 9)
wherein L is Leu, M is Met, Q is Gln, V is Val, Y is Tyr, H is His, P is Pro.

**PREFERRED EMBODIMENTS**

Among the preferred embodiment of the bone implant matrix according to the present invention there are:

1) A bone implant matrix comprising:

a base matrix the surface thereof is coated with a statistically homogeneous composition which is a reinforcing mixture containing at least a soluble polymer, at least a substance, which is able to promote the cell-rooting and the cell growth, by stimulating cell proliferation and tissue integration ("friendliness to cell") and at least one artificial Proline-Rich Peptide wherein the base matrix is selected from the group comprising:

- acellularized or acellularized non-demineralised bone matrix of any source,
- matrix of natural mineral sources,
- synthetic bioceramics matrix,

or mixtures thereof,
the soluble polymer of the reinforcing mixture is a biodegradable polyester or co-polymer thereof, the "friendliness to cell" is selected from the group consisting of gelatine, hydrolysed gelatine and the artificial Proline-Rich Peptide is selected from:

an artificial peptide comprising the amino acid sequence of Pro-X-X-Pro-Y-Y-Y-Pro-X-X-Pro-Y-Y-Pro-X-X-Pro-X-Pro-Y-Y-Y-Y-Y-Y-Pro-Y-Y-Y-Y-Y-Y-Pro-X-X-Pro-X-Pro-Y-Y-Y-Pro-Y-Y-Pro-Y- Pro-X-X-Pro -Y-Pro-Y-Y-Pro-X-X-Pro-Y-Y-Pro-X-X-Pro-Y-Y -Pro-X-X -Pro-Y-Pro-Pro-X-Pro-Pro -X-X-X-X-X-X-X-X-Pro-X-X-Pro-X-X-X-X (SEQ ID NO 1), wherein:

a) Pro is proline;
b) X is an amino acid selected from the group consisting of Ala, Ile, Leu, Met, Phe, Trp and Val, preferably Ile, Leu, Val and Met;
c) Y, is an amino acid selected from the group consisting of Asn, Cys, Gln, Ser, Thr and Tyr, preferably Ser and Gln. or

an artificial peptide comprising the amino acid sequence of Pro-X-X-Pro-Y-Y-Pro-X-X-Pro-Y-Y- Pro-X-X-Pro-Y-Y-Pro-Y-Pro-Pro-X-Pro-Pro (SEQ ID NO 2), wherein:

 a) Pro is proline;
 b) X is an amino acid selected from the group consisting of Ala, Ile, Leu, Met, Phe, Trp and Val;
 c) Y is an amino acid selected from the group consisting of Asn, Cys, Gln, Ser, Thr and Tyr.

2) A bone implant matrix according to embodiment 1, wherein the bio-degradable polyester or copolymer thereof is selected from the group consisting of polylactic acid (PLA), polyglycolic acid (PGA), polycaprolactone (PCL) and co-polymers thereof comprising polycaprolactone-polylactic (PLA/PCL) co-polymers, poly(L-lactide-co- -caprolactone) co-polymers, poly(L-lactide), poly(D,L-lactide-co-glycolide), poly(L-lactide-co-D,L-lactide), their enantiomers, their co-polymers and mixtures thereof.

3) A bone implant matrix according to embodiment 1), wherein the biodegradable polyester or co-polymer thereof is selected from the group consisting of poly(caprolactones), poly(L-lactide), poly(D,L-lactide-co-glycolide), poly(L-lactide-co-D,L-lactide), their enantiomers, their co-polymers and mixtures thereof.

4) A bone implant matrix according to embodiment 1), wherein the biodegradable polyester or co-polymer thereof is selected from the group consisting of polycaprolactone-polylactic copolymer (PLA/PCL) or poly(L-lactide-co- -caprolactone) co-polymer.

5) A bone implant matrix according to embodiment 1), wherein the artificial Proline-Rich Peptide is an artificial peptide comprising the amino acid sequence selected from the group comprising:

 PLV PSY PLV PSY PLV PSY PYP PLPP (SEQ ID NO 3),
 PLV PSQ PLV PSQ PLV PSQ PQP PLPP (SEQ ID NO 4),
 PLV PCC PLV PCC PLV PCC PCP PLPP (SEQ ID NO 5),
 PMM PSY PMM PSY PMM PSY PYP PMPP (SEQ ID NO 6),
 PLV PSS PLV PSS PLV PSS PSP PLPP (SEQ ID NO 7),
 PLV PSS PLV PCC PLV PCC PSP PLPP (SEQ ID NO 8),
 PHQ PMQ PQP PVH PMQ PLP PQP PLPP (SEQ ID NO 9)

or a combination thereof.

6) A bone implant matrix according to embodiment 1), wherein the artificial Proline-Rich Peptide is an artificial peptide comprising the amino acid sequence selected from the group comprising:

 PLV PSQ PLV PSQ PLV PSQ PQ PPLPP (SEQ ID NO 4),
 PLV PSS PLV PCC PLV PCC PSP PLPP (SEQ ID NO 8),
 PHQ PMQ PQP PVH PMQ PLP PQ PPLPP (SEQ ID NO 9)

or a combination of the amino acid sequence of:

 PLV PSQ PLV PSQ PLV PSQ PQP PLPP (SEQ ID NO 4) and
 PLV PSS PLV PCC PLV PCC PSP PLPP (SEQ ID NO 8),
 PLV PSQ PLV PSQ PLV PSQ PQP PLPP (SEQ ID NO 4) and
 PHQ PMQ PQP PVH PMQ PLP PQP PLPP (SEQ ID NO 9),
 PLV PSS PLV PCC PLV PCC PSP PLPP (SEQ ID NO 8) and
 PHQ PMQ PQP PVH PMQ PLP PQP PLPP (SEQ ID NO 9).

7) A bone implant matrix according to embodiment 1), wherein the artificial Proline-Rich Peptide is an artificial peptide comprising the amino acid sequence of:
PHQ PMQ PQP PVH PMQ PLP PQP PLPP (SEQ ID NO 9).

8) A bone implant matrix according to embodiment 1), wherein the artificial Proline-Rich Peptide is an artificial peptide comprising the amino acid sequence of:
PLV PSQ PLV PSQ PLV PSQ PQP PLPP (SEQ ID NO 4).

9) A bone implant matrix according to embodiment 1), wherein the artificial Proline-Rich Peptide is a combination of the amino acid sequence of:

 PLV PSQ PLV PSQ PLV PSQ PQP PLPP (SEQ ID NO 4) and
 PHQ PMQ PQP PVH PMQ PLP PQP PLPP (SEQ ID NO 9).

10) A bone implant matrix according to embodiment 1), wherein the acellularized or acellularized non-demineralised bone matrix of any source is elected from the group comprising:

- acellularized or acellularized non-demineralised human-derived bone matrix,
- acellularized or acellularized non-demineralised xeno-derived bone matrix, such as acellularized or acellularized non-demineralised bovine-bone matrix, acellularized or acellularized non-demineralised equine-bone matrix, acellularized or acellularized non-demineralised porcine-bone matrix.

11) A bone implant matrix according to embodiment 1), wherein the matrix of natural mineral sources is selected from the group comprising mother of pearl, coral, nacre.

12) A bone implant matrix according to embodiment 1), wherein the synthetic bioceramics matrix is selected from the group comprising hydroxyl-apatites, calcium carbonates, calcium phosphates, silicon oxides, titanium oxides, aluminium oxides, zirconia oxides, graphites, bioglasses.

13) A bone implant matrix according to embodiment 1), comprising an acellularised bovine bone matrix, coated with a reinforcing mixture comprising a biodegradable polyester-based copolymer, hydrolysed gelatine, such as bovine or porcine hydrolysed gelatine, or gelatine, such as bovine or porcine gelatine and an artificial Proline-Rich Peptide which is an artificial peptide comprising the amino acid sequence selected from the group comprising:

PLV PSY PLV PSY PLV PSY PYP PLPP (SEQ ID NO 3),
PLV PSQ PLV PSQ PLV PSQ PQP PLPP (SEQ ID NO 4),
PLV PCC PLV PCC PLV PCC PCP PLPP (SEQ ID NO 5),
PMM PSY PMM PSY PMM PSY PYP PMPP (SEQ ID NO 6),
PLV PSS PLV PSS PLV PSS PSP PLPP (SEQ ID NO 7),
PLV PSS PLV PCC PLV PCC PSP PLPP (SEQ ID NO 8),
PHQ PMQ PQP PVH PMQ PLP PQP PLPP (SEQ ID NO 9)

or a combination thereof.

14) A bone implant matrix according to embodiment 1), comprising an acellularised non-demineralised bovine bone matrix, coated with a reinforcing mixture comprising a biodegradable polyester-based copolymer, hydrolysed gelatine, such as bovine or porcine hydrolysed gelatine, or gelatine, such as bovine or porcine gelatine and an artificial Proline-Rich Peptide which is an artificial peptide comprising the amino acid sequence selected form the group comprising:

PLV PSY PLV PSY PLV PSY PYP PLPP (SEQ ID NO 3),
PLV PSQ PLV PSQ PLV PSQ PQP PLPP (SEQ ID NO 4),
PLV PCC PLV PCC PLV PCC PCP PLPP (SEQ ID NO 5),
PMM PSY PMM PSY PMM PSY PYP PMPP (SEQ ID NO 6),
PLV PSS PLV PSS PLV PSS PSP PLPP (SEQ ID NO 7),
PLV PSS PLV PCC PLV PCC PSP PLPP (SEQ ID NO 8),
PHQ PMQ PQP PVH PMQ PLP PQP PLPP (SEQ ID NO 9)

or a combination thereof.

15) A bone implant matrix according to embodiments 13-14), wherein the biodegradable polyester-based co-polymer is a polycaprolactone-polylactic copolymer (PLA/PCL).

16) A bone implant matrix according to embodiments 13-14), wherein the biodegradable polyester-based co-polymer is a poly(L-lactide-co- -caprolactone) co-polymer.

17) A bone implant matrix according to embodiments 13-14), wherein the artificial Proline-Rich Peptide is an artificial peptide comprising the amino acid sequence selected from the group comprising:

PLV PSQ PLV PSQ PLV PSQ PQP PLPP (SEQ ID NO 4),
PLV PSS PLV PCC PLV PCC PSP PLPP (SEQ ID NO 8),
PHQ PMQ PQP PVH PMQ PLP PQP PLPP (SEQ ID NO 9)

or a combination of the amino acid sequence of:

PLV PSQ PLV PSQ PLV PSQ PQP PLPP (SEQ ID NO 4) and
PLV PSS PLV PCC PLV PCC PSP PLPP (SEQ ID NO 8),
PLV PSQ PLV PSQ PLV PSQ PQP PLPP (SEQ ID NO 4) and

PHQ PMQ PQP PVH PMQ PLP PQP PLPP (SEQ ID NO 9),
PLV PSS PLV PCC PLV PCC PSP PLPP (SEQ ID NO 8) and
PHQ PMQ PQP PVH PMQ PLP PQP PLPP (SEQ ID NO 9).

18) A bone implant matrix according to embodiments 13-14), wherein the artificial Proline-Rich Peptide is an artificial peptide comprising the amino acid sequence of:
PHQ PMQ PQP PVH PMQ PLP PQP PLPP (SEQ ID NO 9).

19) A bone implant matrix according to embodiment 1), comprising an acellularised bovine bone matrix coated with a reinforcing mixture comprising a biodegradable polyester-based co-polymer selected from the group comprising: polycaprolactone-polylactic copolymer (PLA/PCL) or poly(L-lactide-co- -caprol actone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of:
PLV PSY PLV PSY PLV PSY PYP PLPP (SEQ ID NO 3).

20) A bone implant matrix according to embodiment 1), comprising an acellularised bovine bone matrix coated with a reinforcing mixture comprising a biodegradable polyester-based co-polymer selected from the group comprising: polycaprolactone-polylactic copolymer (PLA/PCL) or poly(L-lactide-co- -caprol actone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of:
PLV PSQ PLV PSQ PLV PSQ PQP PLPP (SEQ ID NO 4).

21) A bone implant matrix according to embodiment 1), comprising an acellularised bovine bone matrix coated with a reinforcing mixture comprising a biodegradable polyester-based co-polymer selected from the group comprising: polycaprolactone-polylactic copolymer (PLA/PCL) or poly(L-lactide-co- -caprol actone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of:
PLV PCC PLV PCC PLV PCC PCP PLPP (SEQ ID NO 5).

22) A bone implant matrix according to embodiment 1), comprising an acellularised bovine bone matrix coated with a reinforcing mixture comprising a biodegradable polyester-based co-polymer selected from the group comprising: polycaprolactone-polylactic copolymer (PLA/PCL) or poly(L-lactide-co- -caprol actone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of:
PMM PSY PMM PSY PMM PSY PYP PMPP (SEQ ID NO 6).

23) A bone implant matrix according to embodiment 1), comprising an acellularised bovine bone matrix coated with a reinforcing mixture comprising a biodegradable polyester-based co-polymer selected from the group comprising: polycaprolactone-polylactic copolymer (PLA/PCL) or poly(L-lactide-co- -caprol actone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of:
PLV PSS PLV PSS PLV PSS PSP PLPP (SEQ ID NO 7).

24) A bone implant matrix according to embodiment 1), comprising an acellularised bovine bone matrix coated with a reinforcing mixture comprising a biodegradable polyester-based co-polymer selected from the group comprising: polycaprolactone-polylactic copolymer (PLA/PCL) or poly(L-lactide-co- -caprol actone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of:
PLV PSS PLV PCC PLV PCC PSP PLPP (SEQ ID NO 8).

25) A bone implant matrix according to embodiment 1), comprising an acellularised bovine bone matrix coated with a reinforcing mixture comprising a biodegradable polyester-based co-polymer selected from the group comprising: polycaprolactone-polylactic copolymer (PLA/PCL) or poly(L-lactide-co- -caprol actone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of:
PHQ PMQ PQP PVH PMQ PLP PQP PLPP (SEQ ID NO 9).

26) A bone implant matrix according to embodiment 1), comprising an acellularised bovine bone matrix coated with a reinforcing mixture comprising a biodegradable polyester-based co-polymer selected from the group comprising: polycaprolactone-polylactic copolymer (PLA/PCL) or poly(L-lactide-co- -caprol actone) co-polymer, hydrolysed gelatine, such as bovine or porcine hydrolysed gelatine, or gelatine, such as bovine or porcine gelatine and an artificial Proline-Rich Peptide which is an artificial peptide comprising a combination of the amino acid sequence of:

PLV PSQ PLV PSQ PLV PSQ PQP PLPP (SEQ ID NO 4) and
PLV PSS PLV PCC PLV PCC PSP PLPP (SEQ ID NO 8).

27) A bone implant matrix according to embodiment 1), comprising an acellularised bovine bone matrix coated with a reinforcing mixture comprising a biodegradable polyester-based co-polymer selected from the group comprising: polycaprolactone-polylactic copolymer (PLA/PCL) or poly(L-lactide-co- -caprol actone) co-polymer, hydrolysed gelatine, such as bovine or porcine hydrolysed gelatine, or gelatine, such as bovine or porcine gelatine and an artificial Proline-Rich Peptide which is an artificial peptide comprising a combination of the amino acid sequence of:

PLV PSQ PLV PSQ PLV PSQ PQP PLPP (SEQ ID NO 4) and
PHQ PMQ PQP PVH PMQ PLP PQP PLPP (SEQ ID NO 9).

28) A bone implant matrix according to embodiment 1), comprising an acellularised bovine bone matrix coated with a reinforcing mixture comprising a biodegradable polyester-based co-polymer selected from the group comprising: polycaprolactone-polylactic copolymer (PLA/PCL) or poly(L-lactide-co- -caprol actone) co-polymer, hydrolysed gelatine, such as bovine or porcine hydrolysed gelatine, or gelatine, such as bovine or porcine gelatine and an artificial Proline-Rich Peptide which is an artificial peptide comprising a combination of the amino acid sequence of:

PLV PSS PLV PCC PLV PCC PSP PLPP (SEQ ID NO 8) and
PHQ PMQ PQP PVH PMQ PLP PQP PLPP (SEQ ID NO 9).

29) A bone implant matrix according to embodiment 1), comprising an acellularised non-demineralised bovine bone matrix coated with a reinforcing mixture comprising a biodegradable pol-yester-based co-polymer selected from the group com-prising: polycaprolactone-polylactic copolymer (PLA/PCL) or poly(L-lactide-co- -caprolactone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of: PLV PSY PLV PSY PLV PSY PYP PLPP (SEQ ID NO 3).

30) A bone implant matrix according to embodiment 1), comprising an acellularised non-demineralised bovine bone matrix coated with a reinforcing mixture comprising a biodegradable polyester-based co-polymer selected from the group comprising: polycaprolactone-polylactic copolymer (PLA/PCL) or poly(L-lactide-co- -caprolactone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of: PLV PSQ PLV PSQ PLV PSQ PQP PLPP (SEQ ID NO 4).

31) A bone implant matrix according to embodiment 1), comprising an acellularised non-demineralised bovine bone matrix coated with a reinforcing mixture comprising a biodegradable pol-yester-based co-polymer selected from the group com-prising: polycaprolactone-polylactic copolymer (PLA/PCL) or poly(L-lactide-co- -caprolactone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of: PLV PCC PLV PCC PLV PCC PCP PLPP (SEQ ID NO 5).

32) A bone implant matrix according to embodiment 1), comprising an acellularised non-demineralised bovine bone matrix coated with a reinforcing mixture comprising a biodegradable polyester-based co-polymer selected from the group com-prising: polycaprolactone-polylactic copolymer (PLA/PCL) or poly(L-lactide-co- -caprolactone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of: PMM PSY PMM PSY PMM PSY PYP PMPP (SEQ ID NO 6).

33) A bone implant matrix according to embodiment 1), comprising an acellularised non-demineralised bovine bone matrix coated with a reinforcing mixture comprising a biodegradable polyester-based co-polymer selected from the group com-prising: polycaprolactone-polylactic copolymer (PLA/PCL) or poly(L-lactide-co- -caprolactone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of: PLV PSS PLV PSS PLV PSS PSP PLPP (SEQ ID NO 7).

34) A bone implant matrix according to embodiment 1), com-prising an acellularised non-demineralised bovine bone matrix coated with a reinforcing mixture comprising a biodegradable polyester-based co-polymer selected from the group com-prising: polycaprolactone-polylactic copolymer (PLA/PCL) or poly(L-lactide-co- -caprolactone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of: PLV PSS PLV PCC PLV PCC PSP PLPP (SEQ ID NO 8).

35) A bone implant matrix according to embodiment 1), com-prising an acellularised non-demineralised bovine bone matrix coated with a reinforcing mixture comprising a biodegradable polyester-based co-polymer selected from the group com-prising: polycaprolactone-polylactic copolymer (PLA/PCL) or poly(L-lactide-co- -caprolactone) co-polymer, hydrolysed gelatine, such as bovine or porcine hydrolysed gelatine, or gelatine, such as bovine or porcine gelatine and an artificial Proline-Rich Peptide which is an artificial peptide comprising the amino acid sequence of: PHQ PMQ PQP PVH PMQ PLP PQP PLPP (SEQ ID NO 9).

36) A bone implant matrix according to embodiment 1), comprising an acellularised non-demineralised bovine bone matrix coated with a reinforcing mixture comprising a biodegradable polyester-based co-polymer selected from the group comprising: polycaprolactone-polylactic copolymer (PLA/PCL) or poly(L-lactide-co- -caprolactone) co-polymer, hydrolysed gelatine, such as bovine or porcine hydrolysed gelatine, or gelatine, such as bovine or porcine gelatine and an artificial Proline-Rich Peptide which is an artificial peptide comprising a combination of the amino acid sequence of:

PLV PSQ PLV PSQ PLV PSQ PQP PLPP (SEQ ID NO 4) and
PLV PSS PLV PCC PLV PCC PSP PLPP (SEQ ID NO 8).

37) A bone implant matrix according to embodiment 1), comprising an acellularised non-demineralised bovine bone matrix coated with a reinforcing mixture comprising a biodegradable polyester-based co-polymer selected from the group comprising: polycaprolactone-polylactic copolymer (PLA/PCL) or poly(L-lactide-co- -caprolactone) co-polymer, hydrolysed gelatine, such as bovine or porcine hydrolysed gelatine, or gelatine, such as bovine or porcine gelatine and an artificial Proline-Rich Peptide which is an artificial peptide comprising a combination of the amino acid sequence of:

PLV PSQ PLV PSQ PLV PSQ PQP PLPP (SEQ ID NO 4) and
PHQ PMQ PQP PVH PMQ PLP PQP PLPP (SEQ ID NO 9).

38) A bone implant matrix according to embodiment 1), com-prising an acellularised non-demineralised bovine bone matrix coated with a reinforcing mixture comprising a biodegradable pol-yester-based co-polymer selected from the group com-prising: polycaprolactone-polylactic copolymer (PLA/PCL) or poly(L-lactide-co- -caprolactone) co-polymer, hydrolysed gelatine, such as bovine or porcine hydrolysed gelatine, or gelatine, such as bovine or porcine gelatine and an artificial Proline-Rich Peptide belonging to Intrinsic Disorder Protein (IDP) which is an artificial peptide comprising a combination of the amino acid sequence of:

PLV PSS PLV PCC PLV PCC PSP PLPP (SEQ ID NO 8) and
PHQ PMQ PQP PVH PMQ PLP PQP PLPP (SEQ ID NO 9).

39) A bone implant matrix according to embodiment 1), for use in at least one of the following fields: bone reconstructive surgery, in bone regeneration surgery, in regenerative surgery, in skull and maxillo-facial bone reconstructive surgery, in oncologic surgery, pediatric cases, in oral surgery, dental surgery, orthopaedic surgery, spine surgery, traumatology and implantology.

40) A bone implant matrix according to embodiment 1), for use in human or veterinary treatment.

As further preferred embodiments of the present invention there are:

I) a bone implant matrix comprising an acellularised bovine bone matrix the surface thereof is coated with a statistically homogeneous composition which is a reinforcing mixture comprising a biodegradable polyester-based co-polymer, preferably polycaprolactone-polylactic copolymer (PLA/PCL) or poly(L-lactide-co- -caprolactone) co-polymer, hydrolysed gelatine, such as bovine or porcine hydrolysed gelatine, or gelatine, such as bovine or porcine gelatine, and an artificial Proline-Rich Peptide which is an artificial peptide comprising the amino acid sequence selected from the group comprising:

PLV PSY PLV PSY PLV PSY PYP PLPP (SEQ ID NO 3),
PLV PSQ PLV PSQ PLV PSQ PQP PLPP (SEQ ID NO 4),
PLV PCC PLV PCC PLV PCC PCP PLPP (SEQ ID NO 5),
PMM PSY PMM PSY PMM PSY PYP PMPP (SEQ ID NO 6),
PLV PSS PLV PSS PLV PSS PSP PLPP (SEQ ID NO 7),
PLV PSS PLV PCC PLV PCC PSP PLPP (SEQ ID NO 8),
PHQ PMQ PQP PVH PMQ PLP PQP PLPP (SEQ ID NO 9),

preferably an artificial Proline-Rich Peptide which is an artificial peptide comprising the amino acid sequence selected from the group comprising:

PLV PSQ PLV PSQ PLV PSQ PQP PLPP (SEQ ID NO 4),
PLV PSS PLV PCC PLV PCC PSP PLPP (SEQ ID NO 8),
PHQ PMQ PQP PVH PMQ PLP PQP PLPP (SEQ ID NO 9)

or a combination of the amino acid sequence of:

PLV PSQ PLV PSQ PLV PSQ PQP PLPP (SEQ ID NO 4) and
PLV PSS PLV PCC PLV PCC PSP PLPP (SEQ ID NO 8),
PLV PSQ PLV PSQ PLV PSQ PQP PLPP (SEQ ID NO 4) and
PHQ PMQ PQP PVH PMQ PLP PQP PLPP (SEQ ID NO 9),
PLV PSS PLV PCC PLV PCC PSP PLPP (SEQ ID NO 8) and
PHQ PMQ PQP PVH PMQ PLP PQP PLPP (SEQ ID NO 9),

more preferably the artificial Proline-Rich Peptide is an artificial peptide comprising the amino acid sequence of:
PHQ PMQ PQP PVH PMQ PLP PQP PLPP (SEQ ID NO 9).

II) a bone implant matrix comprising an acellularised non-demineralised bovine bone matrix the surface thereof is coated with a statistically homogeneous composition which is a reinforcing mixture comprising a biodegradable polyester-based co-polymer, preferably polycaprolactone-polylactic copolymer (PLA/PCL) or poly(L-lactide-co- -caprolactone) co-polymer, hydrolysed gelatine, such as bovine or porcine hydrolysed gelatine, or gelatine, such as bovine or porcine gelatine, and an artificial Proline-Rich Peptide which is an artificial peptide comprising the amino acid sequence selected from the group comprising:

PLV PSY PLV PSY PLV PSY PYP PLPP (SEQ ID NO 3),
PLV PSQ PLV PSQ PLV PSQ PQP PLPP (SEQ ID NO 4),
PLV PCC PLV PCC PLV PCC PCP PLPP (SEQ ID NO 5),
PMM PSY PMM PSY PMM PSY PYP PMPP (SEQ ID NO 6),
PLV PSS PLV PSS PLV PSS PSP PLPP (SEQ ID NO 7),
PLV PSS PLV PCC PLV PCC PSP PLPP (SEQ ID NO 8),
PHQ PMQ PQP PVH PMQ PLP PQP PLPP (SEQ ID NO 9),

preferably an artificial Proline-Rich Peptide which is an artificial peptide comprising the amino acid sequence selected from the group comprising:

PLV PSQ PLV PSQ PLV PSQ PQP PLPP (SEQ ID NO 4),
PLV PSS PLV PCC PLV PCC PSP PLPP (SEQ ID NO 8),
PHQ PMQ PQP PVH PMQ PLP PQP PLPP (SEQ ID NO 9)

or a combination of the amino acid sequence of:

PLV PSQ PLV PSQ PLV PSQ PQP PLPP (SEQ ID NO 4) and
PLV PSS PLV PCC PLV PCC PSP PLPP (SEQ ID NO 8),
PLV PSQ PLV PSQ PLV PSQ PQP PLPP (SEQ ID NO 4) and
PHQ PMQ PQP PVH PMQ PLP PQP PLPP (SEQ ID NO 9),
PLV PSS PLV PCC PLV PCC PSP PLPP (SEQ ID NO 8) and
PHQ PMQ PQP PVH PMQ PLP PQP PLPP (SEQ ID NO 9),

more preferably the artificial Proline-Rich Peptide is an artificial peptide comprising the amino acid sequence of:
PHQ PMQ PQP PVH PMQ PLP PQP PLPP (SEQ ID NO 9).

The bone implant matrixes described above may be used in orthopedic, oncologic, pediatric, spine and oral surgery, in neurosurgery related to skull bones, in bone reconstructive surgery in general and in implantology.
Said bone implant matrixes are particularly suitable to be used in all bone reconstructive surgery, in particular to reconstruct and missing bone structures due e.g. to trauma-related defects, oncologic resection and/or curettage procedures of bone cysts of different origins, any bone loss due to either external or internal causes or pathologies or any metabolic disorders.
According to a preferred usage, said bone implant matrixes are particularly suitable in the bone reconstructive surgery, following the resection of pathologic bone segments, curettage of bone cysts.
Additionally, said bone implant matrixes may be used also in oral and dental applications, dentistry, as bone "chips", as support matrixes for cell housing and in cellular therapies.

The bone implant matrixes may be used for both human and veterinary use.

It is a further object of the present invention a bone implant matrix herewith disclosed, for use in at least one of the following fields: bone reconstructive surgery, bone regeneration surgery, regenerative surgery, skull and maxillo-facial bone reconstructive surgery, oncologic surgery, pediatric cases surgery, oral surgery, dental surgery, orthopaedic surgery, spine surgery, traumatology and implantology.

It is a further object of the present invention a bone implant matrix herewith disclosed, for use in in human or veterinary treatment.

According to the present invention, a bone implant matrix herewith disclosed may be used for the *in vivo* induction and/or stimulation of biomineralization, such as *in vivo* induction of bone, cartilage, cementum and/or dental tissue formation and/or regeneration.

The present invention also relates to a method for the *in vivo* induction and/or stimulation of biomineralization, such as *in vivo* induction of bone, and/or regeneration, of a bone implant matrix in a subject, for bone reconstructive surgery, in bone regeneration surgery, in regenerative surgery, in maxillo-facial bone reconstructive surgery, in oral surgery, dental surgery, orthopaedic surgery, spine surgery, traumatology, oncological bone reconstructive surgeries and implantology, said method comprising the steps of:

> I) providing a bone implant matrix which comprises:

>> a base matrix the surface thereof is coated with a statistically homogeneous composition which is a reinforcing mixture containing at least a polymer, at least a substance, which is able to promote the cell-rooting and the cell growth, by stimulating cell proliferation and tissue integration ("friendliness to cell") and at least an artificial Proline-Rich Peptide wherein the base matrix is selected from the group comprising:

>>> - acellularized or acellularized non-demineralised bone matrix of any source,
>>> - acellularized or acellularized non-demineralised bone matrix of xeno-origin;
>>> - matrix of natural mineral sources,
>>> - synthetic bioceramics matrix,

>> or mixtures thereof,

>> the polymer of the reinforcing mixture is a biodegradable polyester or co-polymer thereof, the "friendliness to cell" is selected from the group consisting of gelatine, hydrolysed gelatine and the artificial Proline-Rich Peptide is selected from: an artificial peptide comprising the amino acid sequence of Pro-X-X-Pro-Y-Y-Y-Pro-X-X-Pro-Y-Y-Pro-X-X-Pro-X-Pro-Y-Y-Y-Y-Y-Y-Pro-Y-Y-Y-Y-Y-Pro-X-X-Pro-X-Pro-Y-Y-Y-Pro-Y-Y-Pro-Y- Pro-X-X-Pro-Y-Pro-Y-Y-Pro-X-X-Pro-Y-Pro-X-X-Pro-Y-Y-Pro-X-X-Pro-Y-Pro-Pro-X-Pro-Pro-X-X-X-X-X-X-X-X-Pro-X-X-Pro-X-X-X-X (SEQ ID NO 1), wherein:

>>> a) Pro is proline;
>>> b) X is an amino acid selected from the group consisting of Ala, Ile, Leu, Met, Phe, Trp and Val, preferably Ile, Leu, Val and Met;
>>> c) Y, is an amino acid selected from the group consisting of Asn, Cys, Gln, Ser, Thr and Tyr, preferably Ser and Gln. or

>> an artificial peptide comprising the amino acid sequence of Pro-X-X-Pro-Y-Y-Pro-X-X-Pro-Y-Y- Pro-X-X-Pro-Y-Y-Pro-Y-Pro-Pro-X-Pro-Pro (SEQ ID NO 2), wherein:

>>> a) Pro is proline;
>>> b) X is an amino acid selected from the group consisting of Ala, Ile, Leu, Met, Phe, Trp and Val;
>>> c) Y is an amino acid selected from the group consisting of Asn, Cys, Gln, Ser, Thr and Tyr;

> II) implanting said bone implant matrix into said subject.

In order to achieve this, a bone implant matrix according to the invention is administered to the tissue of interest. The bone implant matrix may be administered in any suitable way depending on the intended use. When the bone implant matrix of the invention is administered to a tissue it may induce biomineralization and further bone formation. Examples of tissue of interest in the present context include bone, cartilage, cementum and teeth. Examples of conditions that may lead to bone fractures include, but are not limited to, bone resection, e.g. at trauma, tumours, cysts, and infections or inflammations, such as periodontitis, periimplantitis or ostitis. The bone implant matrix according to the invention may be administered to a subject in need thereof suffering from such a condition.

It is a further object of the present invention a method for the in vivo induction of bone, cartilage, cementum and/or dental tissue formation comprising the administration of a bone implant matrix comprising:

a base matrix the surface thereof is coated with a statistically homogeneous composition which is a reinforcing mixture containing at least a soluble polymer, at least a substance, which is able to promote the cell-rooting and the cell growth, by stimulating cell proliferation and tissue integration ("friendliness to cell") and at least an artificial Proline-Rich Peptide wherein the base matrix is selected from the group comprising:

-   acellularized or acellularized non-demineralised bone matrix of any source,
-   acellularized or acellularized non-demineralised bone matrix of xeno-origin;
-   matrix of natural mineral sources,
-   synthetic bioceramics matrix,

or mixtures thereof,
the soluble polymer of the reinforcing mixture is a biodegradable polyester or co-polymer thereof, the "friendliness to cell" is selected from the group consisting of gelatine, hydrolysed gelatine and the artificial Proline-Rich Peptide is selected from: an artificial peptide comprising the amino acid sequence of Pro-X-X-Pro-Y-Y-Y-Pro-X-X-Pro-Y-Y-Pro-X-X-Pro-X-Pro-Y-Y-Y-Y-Y-Pro-Y-Y-Y-Y-Y-Pro-X-X-Pro-X-Pro-Y-Y-Pro-Y-Y-Pro-Y- Pro-X-X-Pro -Y-Pro-Y-Y-Pro-X-X-Pro-Y-Y-Pro-X-X-Pro-Y-Y -Pro-X-X -Pro-Y-Pro-Pro-X-Pro-Pro -X-X-X-X-X-X-X-Pro-X-X-Pro-X-X-X-X (SEQ ID NO 1), wherein:

a) Pro is proline;
b) X is an amino acid selected from the group consisting of Ala, Ile, Leu, Met, Phe, Trp and Val, preferably Ile, Leu, Val and Met;
c) Y, is an amino acid selected from the group consisting of Asn, Cys, Gln, Ser, Thr and Tyr, preferably Ser and Gln. or

an artificial peptide comprising the amino acid sequence of Pro-X-X-Pro-Y-Y-Pro-X-X-Pro-Y-Y- Pro-X-X-Pro-Y-Y-Pro-Y-Pro-Pro-X-Pro-Pro (SEQ ID NO 2), wherein:

a) Pro is proline;
b) X is an amino acid selected from the group consisting of Ala, Ile, Leu, Met, Phe, Trp and Val;
c) Y is an amino acid selected from the group consisting of Asn, Cys, Gln, Ser, Thr and Tyr, to a subject in need thereof.

A method for preparing the bone implant matrixes is be described herein below, which comprises the following steps:

a) preparing a solution of a reinforcing mixture containing at least a soluble polymer, at least a substance, which is able to promote the cell-rooting and the cell growth, by stimulating cell proliferation and tissue integration ("friendliness to cell") and at least an artificial Proline-Rich Peptide, as described above,
b) immersing a base matrix, according to the present invention, into the reinforcing mixture made according to step a),
c) drying and degassing the matrix made according to step b), preferably in a vacuum furnace at 37°C ($\pm$2°C) for 24 hours, for removing possible solvent residues (for example in air or preferably in a vacuum furnace).

Drying and degassing the bone implant matrix usually take place contemporarily.

Such a method may optionally be followed by a post-treatment step, which comprises for example heating, conditioning in an inert atmosphere the bone implant matrixes and degassing to remove completely the possible residues of solvents used in the preparation process.

Moreover, the bone implant matrix preparation process may be followed by a packaging method which comprises the steps of:

d) packaging in a sterile and inert atmosphere,
e) sterilization (preferably through ethylene oxide or beta-ray irradiation).

The matrixes known in the state of the art and commonly used in the orthopaedic surgery have poor mechanical resistance, poor fixing resistance, fragility and little ductility characteristics.

The Applicant has surprisingly found that, by treating a base matrix, according to the present invention, with a reinforcing mixture containing at least a soluble polymer, at least a substance, which is able to promote the cell-rooting and the cell growth, by stimulating cell proliferation and tissue integration ("friendliness to cell") and at least an artificial Proline-Rich Peptide, as described above, it is possible to obtain bone implant matrixes, which have

The following, not limitative, examples describe embodiments of the invention.

## EXAMPLES

## EMBODIMENTS ACCORDING TO THE INVENTION

In order to show the feasibility, reproducibility and efficacy of the bone implant matrix "SBP" according to the present invention, different types of base matrixes, soluble polymers, "friendliness to cell" and artificial Proline-rich peptides, also defined as "PRP", according to the present invention, have been combined according to the following examples 2A - 2E and the corresponding efficacy have been tested, ideally targeting a 1$\mu$g of peptide per 1cc of base matrix.

## Example 1: Preparation of peptide solutions

For each of the three peptide: PLV PSQ PLV PSQ PLV PSQ PQP PLPP (SEQ ID NO 4), PLV PSS PLV PCC PLV PCC PSP PLPP (SEQ ID NO 8) and PHQ PMQ PQP PVH PMQ PLP PQ PPLPP (SEQ ID NO 9) were prepared the corresponding stock solutions at 4 mM in 0.1% w/w acetic acid (in water - sterile filtered. Make aliquots and store at - 20°C.

## Example 2A: Method for preparing a bone implant matrix with artificial Proline-rich Peptide PHQ PMQ PQP PVH PMQ PLP PQ PPLPP (SEQ ID NO 9)

Bring into solution 1 g of poly(L-lactide-co- -caprolactone) co-polymer (PLCL) in 20 ml of dichloromethane. Keep under stirring with a magnetic stirrer for at least 45 minutes at room temperature, in order to obtain a solution having a very homogeneous dispersion.

Prepare 20 ml of 1.5% w/w aqueous solution of hydrolysed porcine gelatine. Pour water, preferably by injection, and gently stirring, add the hydrolysed porcine gelatine. Keep under stirring for at least 1 hour at 37°C ($\pm$ 3°C), in order to obtain a solution characterised by a very homogeneous dispersion.

Add 10 ml of isopropanol to the poly(L-lactide-co- -caprolactone) co-polymeric solution in dichloromethane previously prepared.

Keep the obtained polymeric solution under stirring for 15 minutes.

Add to the hydrolysed porcine gelatine aqueous solution, previously prepared, the peptide PHQ PMQ PQP PVH PMQ PLP PQ PPLPP (SEQ ID NO 9) adding the solution thereof in order to achieve a final concentration not less than 0.025 $\mu$M of said peptide.

Add the hydrolysed porcine gelatine/peptide solution, previously prepared, to the co-polymeric solution.

Keep the final co-polymeric solution so obtained well stirred for 10 minutes at room temperature, in order to obtain a stable, well homogeneous and nano-dispersed solution of all the compounds used.

Immerse the non-demineralised, acellularized bovine bone matrix into the polymeric solution and keep immersed for at least 30 minutes under stirring.

At the end, insert the product in the vacuum furnace for 24 hours at 37°C ($\pm$ 3°C) in order to remove the solvents.

## Example 2B. Method for preparing a bone implant matrix with artificial Proline-rich Peptide PLV PSQ PLV PSQ PLV PSQ PQP PLPP (SEQ ID NO 4) identified as P2 with High concentration thereof: SBP2H

Bring into solution 1 g of poly(L-lactide-co- -caprolactone) co-polymer (PLCL) in 20 ml of dichloro-methane. Keep under stirring with a magnetic stirrer for at least 45 minutes at room temperature, in order to obtain a solution characterised by a very homogeneous dispersion.

Prepare 20 ml of 1.5% w/w aqueous solution of hydrolysed porcine gelatine. Pour water, preferably by injection, and gently stirring, add the hydrolysed porcine gelatine. Keep under stirring for at least 1 hour at 37°C ($\pm$ 3°C), in order to obtain a solution characterised by a very homogeneous dispersion.

Add 10 ml of isopropanol to the poly(L-lactide-co- -caprolactone) co-polymeric solution in dichloro-methane previously prepared.

Keep the obtained polymeric solution under stir-ring for 15 minutes.

Add to the hydrolysed porcine gelatine aqueous solution, previously prepared, the peptide PLV PSQ PLV PSQ PLV PSQ PQP PLPP (SEQ ID NO 4) adding the solution thereof in order to achieve a final concentration not less than 0.025 μM of said peptide.

Add the hydrolysed porcine gelatine/peptide solution, previously prepared, to the co-polymeric solution.

Keep the final co-polymeric solution so obtained well stirred for 10 minutes at room temperature, in order to obtain a stable, well homogeneous and nano-dispersed solution of all the com-pounds used.

Immerse the non-demineralised, acellularized bovine bone matrix into the polymeric solution and keep immersed for at least 30 minutes under stirring.

At the end, insert the product in the vacuum furnace for 24 hours at 37°C ($\pm$ 3°C) in order to remove the solvents.

**Example 2C. Method for preparing a bone implant matrix with artificial Proline-rich Peptide PLV PSQ PLV PSQ PLV PSQ PQP PLPP (SEQ ID NO 4) identified as P2 with medium concentration thereof: SBP2M**

Bring into solution 1 g of PLA/PCL co-polymer in 20 ml of dichloro-methane. Keep under stirring with a magnetic stirrer for at least 45 minutes at room temperature, in order to obtain a solution characterised by a very homogeneous dispersion.

Prepare 20 ml of 1.5% w/w aqueous solution of hydrolysed bovine gelatine. Pour water, preferably by injection, and gently stirring, add the hydrolysed bovine gelatine. Keep under stirring for at least 1 hour at 37°C ($\pm$ 3°C), in order to obtain a solution characterised by a very homogeneous dispersion.

Add 10 ml of isopropanol to the PLA/PCL co-polymeric solution in dichloro-methane previously prepared.

Keep the obtained polymeric solution under stir-ring for 15 minutes.

Add to the hydrolysed bovine gelatine aqueous solution, previously prepared, the peptide PLV PSQ PLV PSQ PLV PSQ PQP PLPP (SEQ ID NO 4) adding the solution thereof in order to achieve a final concentration not higher than 0.025 μM of said peptide.

Add the hydrolysed bovine gelatine/peptide solution, previously prepared, to the co-polymeric solution.

Keep the final co-polymeric solution so obtained well stirred for 10 minutes at room temperature, in order to obtain a stable, well homogeneous and nano-dispersed solution of all the com-pounds used.

Immerse the non-demineralised, acellularized bovine bone matrix into the polymeric solution and keep immersed for at least 30 minutes under stirring.

At the end, insert the product in the vacuum furnace for 24 hours at 37°C ($\pm$ 3°C) in order to remove the solvents.

**Example 2D. Method for preparing a bone implant matrix with artificial Proline-rich Peptide PLV PSQ PLV PSQ PLV PSQ PQP PLPP (SEQ ID NO 4) identified as P2 with Low concentration thereof: SBP2L**

Bring into solution 1 g of P(L,DL)LA [poly(70L-lactide-co-30DL-lactide)] co-polymer in 20 ml of dichloromethane. Keep under stirring with a magnetic stirrer for at least 45 minutes at room temperature, in order to obtain a solution having a very homogeneous dispersion.

Prepare 20 ml of 1.5% w/w aqueous solution of porcine gelatine. Pour water, preferably by injection, and gently stirring, add the porcine gelatine. Keep under stirring for at least 1 hour at 37°C ($\pm$ 3°C), in order to obtain a solution characterised by a very homogeneous dispersion.

Add 10 ml of isopropanol to the PLA/PCL co-polymeric solution in dichloro-methane previously prepared.

Keep the obtained polymeric solution under stirring for 15 minutes.

Add to the porcine gelatine aqueous solution, previously prepared, the peptide PLV PSQ PLV PSQ PLV PSQ PQP PLPP (SEQ ID NO 4) identified as P2 adding the solution thereof in order to achieve a final concentration not higher than 2.5 M of said peptide.

Add the porcine gelatine/peptide solution, previously prepared, to the co-polymeric solution.

Keep the final co-polymeric solution so obtained well stirred for 10 minutes at room temperature, in order to obtain a stable, well homogeneous and nano-dispersed solution of all the compounds used.

Immerse the acellularized bovine bone matrix into the polymeric solution and keep immersed for at least 30 minutes under stirring.

At the end, insert the product in the vacuum furnace for 24 hours at 37°C ($\pm$ 3°C) in order to remove the solvents.

**Example 2E. Method for preparing a bone implant matrix with artificial Proline-rich Peptide PLV PSS PLV PCC PLV PCC PSP PLPP (SEQ ID NO 8)**

Bring into solution 1 g of PLA/PCL co-polymer in 20 ml of dichloro-methane. Keep under stirring with a magnetic stirrer for at least 45 minutes at room temperature, in order to obtain a solution characterised by a very homogeneous dispersion.

Prepare 20 ml of 1.5% w/w aqueous solution of porcine gelatine. Pour water, preferably by injection, and gently stirring, add the porcine gelatine. Keep under stirring for at least 1 hour at 37°C (± 3°C), in order to obtain a solution characterised by a very homogeneous dispersion.

Add 10 ml of isopropanol to the PLA/PCL co-polymeric solution in dichloro-methane previously prepared.

Keep the obtained polymeric solution under stir-ring for 15 minutes.

Add to the porcine gelatine aqueous solution, previously prepared, the peptide PLV PSQ PLV PSQ PLV PSQ PQP PLPP (SEQ ID NO 4) identified as P2 adding the solution thereof in order to a final concentration not less than 0.025 $\mu$M of said peptide.

Add the porcine gelatine/peptide solution, previously prepared, to the co-polymeric solution.

Keep the final co-polymeric solution so obtained well stirred for 10 minutes at room temperature, in order to obtain a stable, well homogeneous and nano-dispersed solution of all the com-pounds used.

Immerse the non-demineralised, acellularized bovine bone matrix into the polymeric solution and keep immersed for at least 30 minutes under stirring.

At the end, insert the product in the vacuum furnace for 24 hours at 37°C (± 3°C) in order to remove the solvents.

**COMPARATIVE EMBODIMENTS**

For comparative purposes, in order to verify the efficacy of the bone implant matrix "SBP" according to the present invention, a bone implant matrix, identified as "SB", according to the PCT application no. PCT/IB2009/007759 has been reproduced (example 3) and tested.

**EXAMPLE 3. Method for preparing a bone implant matrix without artificial Proline-rich Peptide: bone graft substitute or SB (not according to the invention).**

Bring into solution 1 g poly(L-lactide-co- -caprolactone) co-polymer (PLCL) in 20 ml of dichloromethane. Keep under stirring for at least 45 minutes at room temperature in order to obtain a solution having a very homogeneous dispersion.

Prepare 20 ml of 1.5% solution of hydrolysed porcine gelatine. Pour water, preferably by injection, and gently stirring, add the hydrolysed porcine gelatine. Keep under stirring for at least 1 hour at 37°C (± 3°C), in order to obtain a solution characterized by a very homogeneous dispersion.

Add 10 ml of isopropanol to the poly(L-lactide-co- -caprolactone) (PLCL) co-polymeric solution in dichloromethane previously prepared.

Keep the so obtained co-polymeric solution under stirring for 20 minutes.

Add the hydrolysed porcine gelatine solution, previously prepared, to the co-polymeric solution.

Keep the co-polymeric solution so obtained well stirred for 10 minutes at room temperature, in order to obtain a stable, well homogeneous and nano-dispersed solution of all the compounds used.

Immerse the bovine non-demineralised, acellularized bone matrix into the polymeric solution and keep immersed for at least 30 minutes under stirring.

At the end, insert the product in the vacuum furnace for 24 hours at 37°C (± 3°C) in order to remove the solvents.

**Example 4. Comparative peptide: EMD**

A gel-coating primarily based on amelogenin for enamel regeneration produce and sell by Straumann® Emdogain® (EMD) as peptides already used in medical biomineralization, has been used for comparison. Prepare a stock solution at 10 mg/ml in 0.1% w/w acetic acid (in water - sterile filtered). Make aliquots and store at -20°C.

**Example5. Experimental tests**

**Release profile**

To obtain the FITC release profile, 10x10x10 mm$^3$ FITC- bone implant matrix "SBP" blocks, according to the present invention (such as those according to examples 2A - 2E), were immerged in 2 ml purified water. For 4 days the solution was analysed every 24 h using UV-vis, then 48 h after, and finally 30 days after. The water was changed every time to better simulate in vivo conditions, maximizing the drive force. Having a FITC+$H_2$O calibration curve, the absorbance could be related to the concentration. Using a known quantity of water, the mass released could be derived from the measured concentration.

For a proper FITC release profile, also samples of bone implant matrix, identified as "SB", reproduced according to example 3 were studied, since other components of the "SB" is likely to be released, the blank used for the spectroscopy was based on a water + "SB" solution. The same method was used for making the release profile of the proper "SBP", such as those according to examples 2A - 2E, except for that 10ml purified water was used instead of 2ml. To identify the presence of "PRP" in the aqueous solution, FITC molecules were either attached to the C- or N-terminal of the peptide before loading.

**Graft characterization**

The grafts were characterized using analytical methods in correspondence with standard methods adopted from literature [G. Perale, P. Arosio, D. Moscatelli, V. Barri, M. Müller, S. MacCagnan, M. Masi, A new model of resorbable device degradation and drug release: transient 1-dimension diffusional model, Journal of Controlled Release 136(3) (2009) 196-205]. The analytical analysis consisted of extended pressure microscopy (environmental SEM, E/SEM) and with energy dispersion spectroscopy (EDX) pointing at random sports on the graft. The used machine was JEOL 6010-LA SEM using 15kV, at x100 mag. with EDS (Tokyo, Japan). External and internal surfaces were investigated. To obtain photos of the internal surfaces the scaffolds were cut into pieces using a scalpel.

As the peptides composition in the coating, i.e. the reinforcing mixture according to the present invention, is very low [Ca. 0.0005% wt.], the mechanical properties of the bone implant matrix "SBP" according to the present invention, is highly likely to be identical to the bone implant matrix, identified as "SB", as reproduced according to Example 3.

**In vitro cell trials**

The biocompatibility, cytotoxicity and bioactivity of the bone implant matrix "SBP" according to the present invention (such as those according to examples 2A - 2E) was performed through *in vitro* analysis of LDH activity, metabolic activity and mineralization. Discs of 16 mm in diameter and 3 mm in height of the bone implant matrix "SBP" according to the present invention (such as those according to examples 2A - 2E) were used for the studies. Discs had 3 different codes: SBP2, SBP5 and SBP6 related to the different treatments with synthetic peptides according to the examples 2A - 2E and control surfaces and studies were performed blinded.

The cells assessed was murine pre-osteoblastic cell line (MC3T3-E1) obtained from the German Collection of Microorganisms and Cell Cultures (DSMC, Braunschweig, Germany). MC3T3-E1 cells were routinely cultured at 37 °C in a humidified atmosphere of 5% $CO_2$, and maintained in $\alpha$-MEM supplemented with 10% foetal calf serum (FCS) and antibiotics (50 IU penicillin/ml and 50 $\mu$g streptomycin/ml). Cells were subcultured 1:4 before reaching confluence using PBS and trypsin/EDTA. All experiments were performed in the same passage of the MC3T3-E1 cells.

To test the different surfaces and treatments of the bone implant matrix "SBP" according to the present invention (such as those according to examples 2A - 2E), the discs were placed in a 12-well plate and $2x10^5$ cells were seeded on each disc. In order to guarantee a homogenous cell distribution inside the bone implant matrix "SBP" according to the present invention (such as those according to examples 2A - 2E), an agitated seeding method was used [M. Gomez-Florit, M. Rubert, J.M. Ramis, H.J. Haugen, H. Tiainen, S.P. Lyngstadaas, M. Monjo, TiO2 Scaffolds Sustain Differentiation of MC3T3-E1 Cells, J Biomater Tiss Eng 2(4) (2012) 336-344.]. Briefly after adding 1ml of cell suspension to the bone implant matrix "SBP" according to the present invention, plates were agitated on an orbital shaker (Unitron, Infors HT, Basel, Switzerland) for 6h at 180rpm at 37 °C and in humidity conditions. Then, cells were maintained static at 37 °C in a humidified atmosphere of 5% $CO_2$ for up to 14 days. The same number of cells were cultured in parallel in plastic in all the experiments.

For the experiments, MC3T3-E1 cells were maintained for 48h (Toxicity, metabolic activity, gene expression, scanning electron microscopy and confocal microscopy) and 2 weeks (gene expression analyses, ALP activity, SEM and confocal microscopy) on the bone implant matrix "SBP" according to the present invention, in $\alpha$-MEM supplemented with 10% FCS and antibiotics.

**Cytotoxicity test:**

Lactate dehydrogenase (LDH) activity, an indicator of cytotoxicity, was measured in culture media after 48 h of incubation with the test samples and controls. A LDH-kit was used. Low control (0% toxicity) was obtained from culture media of MC3T3-E1 cells seeded on plastic (TCP). High control (100 % toxicity) was obtained from culture media of MC3T3-E1 cells seeded on TCP and treated with 1% SDS. The percentage cytotoxicity was found using the equation:

$$\text{Cytotoxicity (\%)} = (\text{Exp.value-low control})/(\text{high control-low control}) \; .$$

**Metabolic activity:**

Total metabolic activity was determined at 2, 6, 14 days of MC3T3-E1 cell culture, using Presto Blue reagent (Life Technologies, Carlsbad, CA). The bone implant matrix "SBP" according to the present invention (such as those according to examples 2A - 2E) with cells were transferred to new tissue culture plates and 100 μl of Presto Blue was added to cells with 1000 μl of culture media. The absorbance of the media was read at 570 and 600nm after 1h of reagent incubation at 37 °C, following manufacturer's protocol.

## Immunoflourescence:

Cells grown for 48h or 14 days on the bone implant matrix "SBP" according to the present invention, were fixed for 15 min with 4 % formaldehyde in PBS at room temperature. For actin cytoskeleton visualization, cells were stained with phalloidin-fluorescein isothiocyanate (phalloidin-FITC) 5 μg/mL (Sigma, St. Louis, MO, USA) in PBS with Triton X-100 0.2 % for 30 min. Then, a drop of Fluoroshield™ with DAPI (Sigma, St. Louis, MO, USA) was added and cover glasses were mounted on the samples.

Two samples of each group were used to perform the experiment and two images of each sample are taken with the confocal microscope (Leica DMI 4000B equipped with Leica TCS SPE laser system).

## Scanning Electron Microscopy:

A visual representation of the seeded cells on superficial surfaces was made using SEM (Hitachi S-3400N, Hitachi High-Technologies Europe GmbH, Krefeld, Germany) at 10kV, 40 Pa, 48h and 14 days after seeding. The cells were fixed with glutaraldehyde in PBS for 2 h. The fixation solution was removed, and the cells were washed with distilled water two times. At 30 min intervals, the cells were dehydrated by the addition of 50%, 70%, 90% and 100% ethanol solutions. Finally, the ethanol was removed, and the cells were left at room temperature to evaporate the remaining ethanol prior to analysis. Samples were cut in half to evaluate cellular invasion in the bone implant matrix "SBP" material according to the present invention. Two samples of each group were used to perform the experiment and two images of each samples were taken with the SEM.

## Real-time PCL analysis:

Total RNA was isolated with Tripure (Roche Diagnostics) and RNA was quantified using a spectrophotometer set at 260 nm. Real-time PCR was performed for two reference genes and several target gene (Table)
The same amount of total RNA from each sample (370 ng) was reverse transcribed to cDNA at 37 °C for 60 min in a final volume of 20 μl, using High Capacity RNA to cDNA kit (Applied Biosystems). Each cDNA was diluted 1/7 and these dilutions are used to carry on the quantitative PCR.

Real-time PCR was performed in the Lightcycler 480® (Roche Diagnostics, Germany) using SYBR green detection. For each reaction, 7 μl of Lightcycler-FastStart DNA MasterPLUS SYBR Green I, 0.5 μM of sense and antisense specific primer and 3 μl of the cDNA dilution in a final volume of 10 μl was added. The normal amplification program consisted of a preincubation step for denaturation of the template cDNA (95 °C), followed by 45 cycles consisting of a denaturation step (95 °C), an annealing step (60 °C, for all expect for ALP and osterix, which were 65 °C and 68 °C respectively) and an extension step (72 °C). Real-time efficiencies were calculated from the given slopes in the LightCycler 480 software using serial dilutions.

Relative quantification after PCRWas calculated by dividing the concentration of the target gene in each sample by the mean of the concentration of the two reference genes (Housekeeping genes) in the same sample using the advanced relative quantification method provided by the LightCycler 480 analysis software version 1.5 (Roche Diagnostics, Mannheim, Germany).

## Alkaline Phosphate Activity

ALP activity was determined from cells after 14 days of cell culture. Cells were washed twice in PBS, solubilized with 0.1% Triton X-100. Then, samples were incubated with an assay mixture of p-Nitrophenyl Phosphate (pNPP). Cleavage of pNPP (Sigma, Saint Louis, Missouri, USA) in a soluble yellow end product which absorb at 405 nm was used to assess ALP activity. In parallel to the samples, a standard curve with calf intestinal alkaline phosphatase (CIAP) (Promega, Madison, USA) was constructed; 1μl from the stock CIAP was mixed with 5 ml of alkaline phosphatase buffer (1:5000 dilution), and subsequently diluted 1:5.

Analytical investigation

**Release profile:**

Calibration was used to derive the concentration of FITC released in the water solution at the given time points. With a known quantity of water used at a given time, the mass released can be derived.

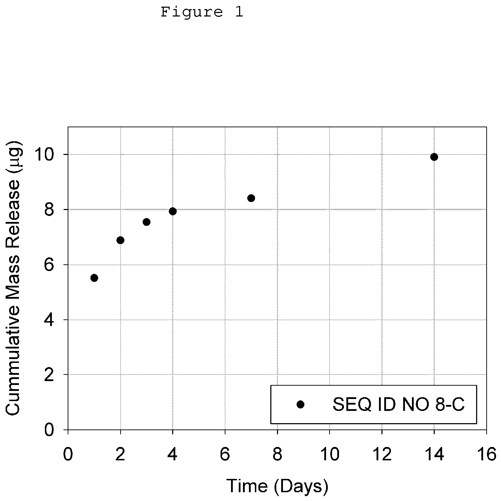

It may be observed Figure 1 that the initial mass release rate is very high (approx. 6$\mu$g the first 24h), before it is quickly dropping. After 36 days, the release rate has dropped significantly indicating that most of the coating has been released. A logarithmic best-fit plot was made to describe the trend, a $R^2$ value of 0.956 suggests that the equation 1.2 is a good fit:

$$Released\ mass = 0.7836 * \ln(t) + 6.2617\ \mu g \qquad 1.2$$

Where 't' is the time in days.

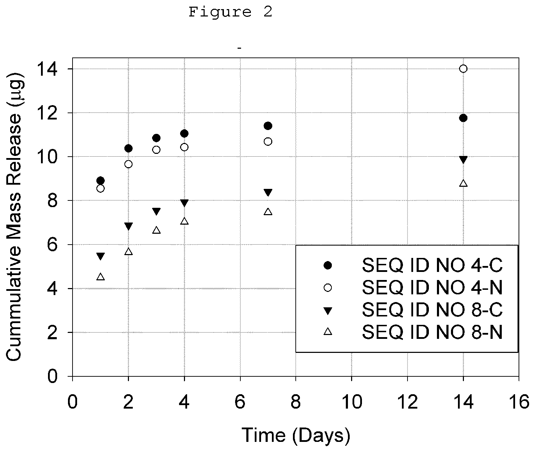

Similar analysis was done for the *PRP* (version P2 and P5, with FITC attached on the C or the N terminals). The experimental data is presented in Figure 2. It may observe that -C and -N groups, of both the two peptide versions, has almost identical release. Only divergence from this trend is after 14 days where the P2N has a jump in release rate, contradictory to the decreasing rate trend. It may also be noticed that the P2 groups has a higher initial release than P5, but the rate drops quicker as well.

Best fit plots where made using the inbuild best-fit function of Graph (Software) assuming both a logarithmic and power function. In general, the logarithmic functions were more accurate ($R^2$-values closer to 1).

**Graft characterization:**

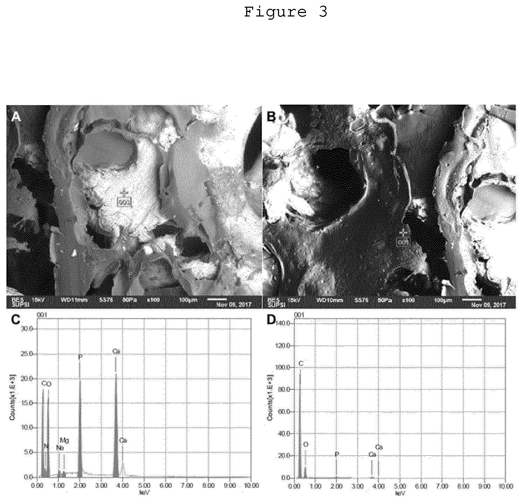

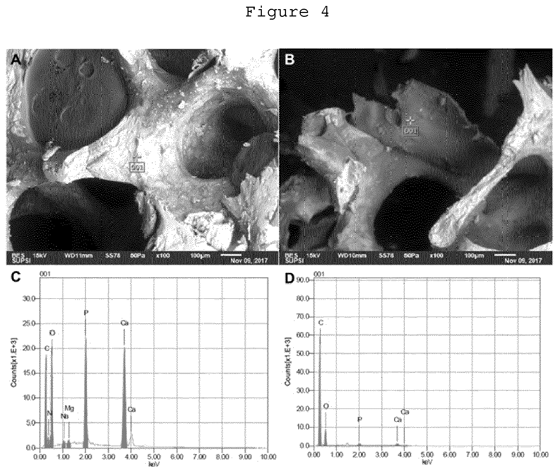

SEM photos and EDS analysis for the given points are found in Figure 3 and 4. When comparing to results from previous literature [G. Pertici, F. Rossi, T. Casalini, G. Perale, Composite polymer-coated mineral grafts for bone regeneration: material characterisation and model study, Annals of Oral & Maxillofacial Surgery 2(1) (2014)], the ESEM study confirmed an identical microstructure for bone implant matrix, identified as "SB", reproduced according to example 3 and the bone implant matrix "SBP" according to the present invention (such as those according to examples 2A - 2E). The polymer phase of the reinforcing mixture is recognized by the very high carbon content (Derived from EDS) compared to the bone phase, the base matrix (approx. 4-5 times as high atom count). Likewise, the bone phase has approx. 10-20 times as much calcium and phosphorous content as the polymer phase. Bone largely consists of crystalline apatite [K. Chatzipanagis, M. Iafisco, T. Roncal-Herrero, M. Bilton, A. Tampieri, R. Kröger, J.M. Delgado-Lopez, Crystallization of citrate-stabilized amorphous calcium phosphate to nanocrystalline apatite: a surface-mediated transformation, CrystEngComm 18(18) (2016) 3170-3173.], which has a molecular structure consisting both of calcium and phosphorous. This confirms that the reinforcing mixture was successfully applied.

From Figure 3 and 4 it may be observed equivalent results on the internal surfaces as the external, indicating a good distribution of the reinforcing mixture coating.

**In vitro results**

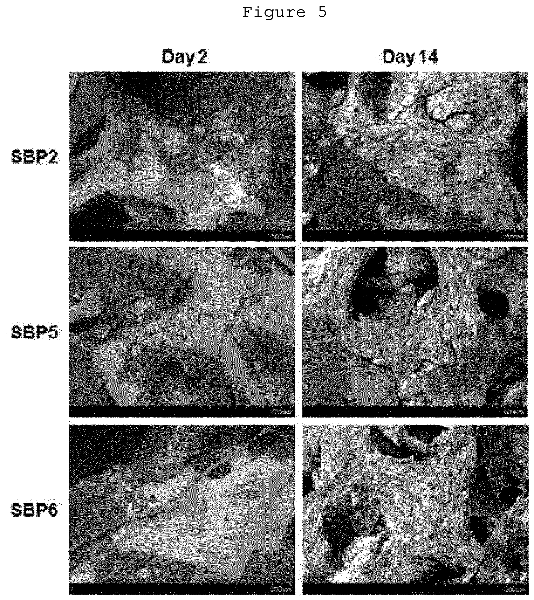

The MC3T3-E1 was successfully seeded on the "SBP" bone graft substitutes, i.e. the bone implant matrix "SBP" according to the present invention (such as those according to examples 2A - 2E). This is visually represented through the SEM photos in Fig 5. There it may be observed considerable morphological changes of the seeded cells on the surface of the graft. After two days the cells are barely observable on the graft surface, however after 14 days there has been excellent cell growth for all groups, and particularly for SBP2.

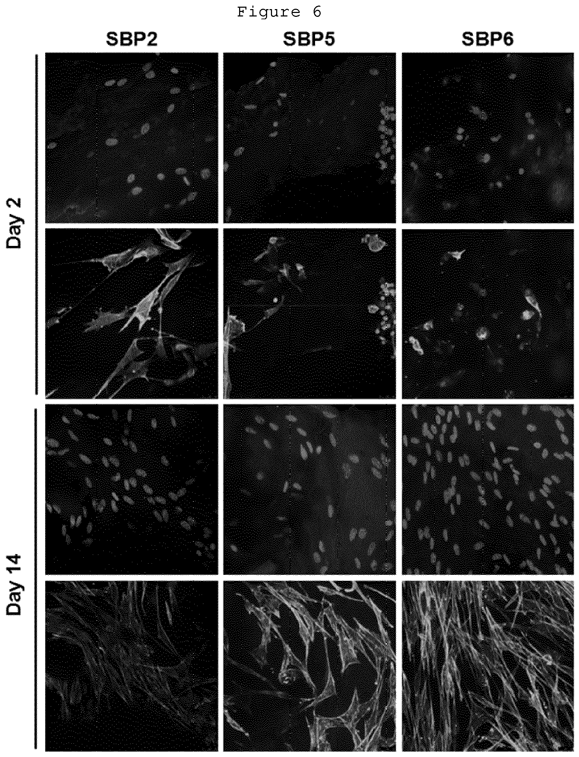

Additionally, cell invasion was investigated using confocal microscopy of stained samples (Figure 6). After two days of culture, although few cells were observed on the SEM images, confocal microscopy confirmed their presence and penetration through the SBP. This was probably due to the attachment and growth of the cells onto the polymer of the reinforcing mixture of the "SBP" samples, i.e. the bone implant matrix "SBP" according to the present invention (such as those according to examples 2A - 2E), which impairs their visualization under the SEM. After 14 days of culture, cells grew well onto the surface of all the "SBP" groups, but few cells could be observed inside the scaffolds by SEM analysis. However, confocal images confirmed the presence of cells through at least 60$\mu$m in depth showing that cells seeded on the SBP2 group penetrated more deeply (pink nuclei can be observed both at 2 and 14 days) compared to SBP5 and SBP6 (no pink nuclei can be observed). These results agree with the increased metabolic activity observed in the SBP2 group, based on the reduction of resazurin to resorufin by metabolically active cells, which correlates with cell proliferation.

**Cytotoxicity:**

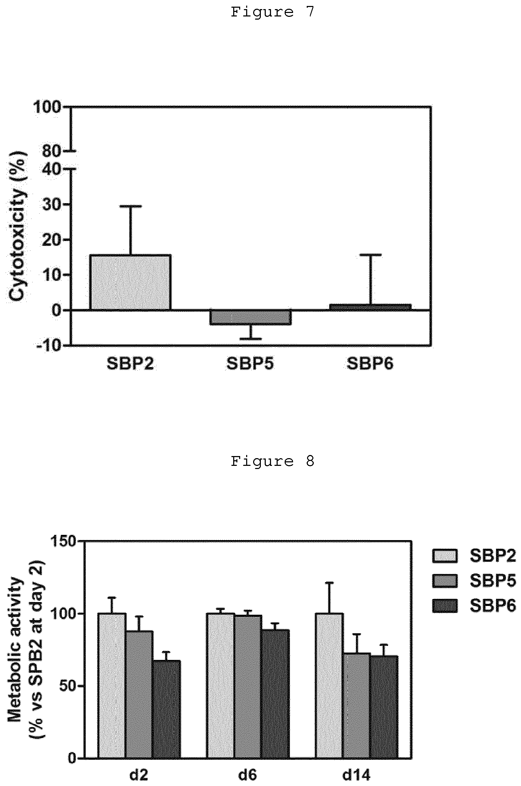

The biocompatibility of the different groups of SBP was first evaluated quantitatively utilizing LDH activity (Figure 7). The LDH assay detects the amount of LDH that leaks out through the plasma membrane of damaged cells, as a marker of cytotoxicity. The tests show low or even negative percentage LDH-levels compared to the controls. This could be translated that none of the "SBP" groups, i.e. the groups of the bone implant matrix "SBP" according to the present invention (such as those according to examples 2A - 2E) had toxicity towards MC3T3-E1 cells after 48h. SBP2 showed the highest level of cytotoxicity, but the mean was still well below 20%. SBP6 showed barely a few percent, meanwhile SBP5 had a negative result. Although One-way ANOVA and Bonferroni post-hoc analyses were performed, there was observed no statistical differences among the 3 groups. Hence, all had similar or lower levels than the control (0% toxicity). All groups of the bone implant matrix "SBP" according to the present invention (such as those according to examples 2A - 2E), had toxicity below 30%, which is the maximum value accepted for cytotoxicity of medical devices according to ISO-10993:5.

**Metabolic Activity:**

The metabolic activity was measured 2, 6 and 14 days after seeding, using SBP2 at day 2 as the basis for the comparison (Figure 8). A higher metabolic activity is an indication of better cell proliferation. After two days, SBP2 had a higher metabolic activity than SBP5 and SBP6. Particularly SBP6 was a lot lower. After 6 days the metabolic activity was much of the same, however after 14 days did SBP2 showed a higher activity again.

**Cell Proliferation:**

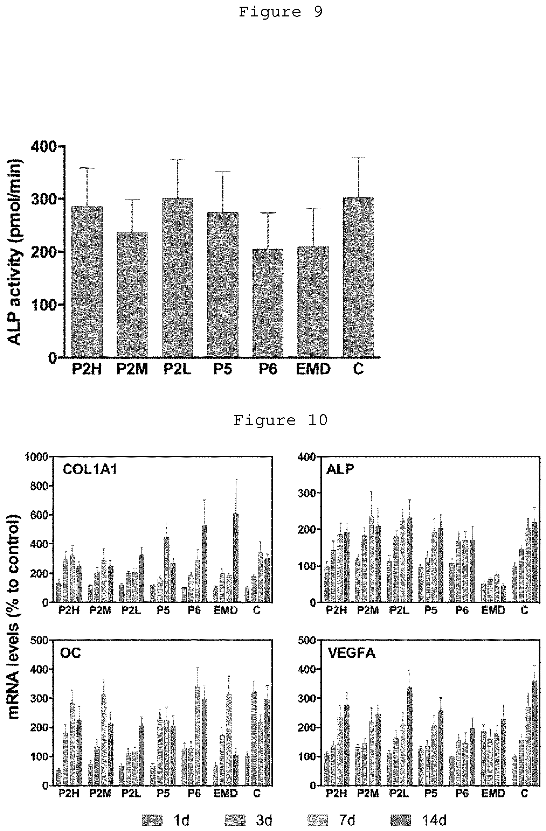

Alkaline phosphate levels were used as indication of cell proliferation. As seen in Figure 9 does SBP6 EMD and, to part, SBP2M have a lower ALP activity, thus a lower proliferation than the remaining groups. The control group has the highest ALP activity, however P2L's level is almost identical and P2H is slightly lower. There was no statistical difference between the groups, meaning that there is a good cell proliferation for all groups.

**Gene Expression:**

The effect of the peptides and EMD on the mRNA levels of different osteogenic markers was measured by real-time PCR after 1, 3, 7 and 14 days in the cell culture. Four different markers were analysed (Figure 10), including collagen type 1 Alpha 1 chain (COL1A1), alkaline phosphate (ALP), osteocalcin (OC) and vascular endothelial growth factor A (VEGFA). All groups showed better mRNA levels, for all gene expressions, than the control group, with exception for the OC levels after one day. This indicates that they all improve biological performance.

For the first few days, the groups all showed similar COL1A1 mRNA levels, before the levels of P6 and particularly EMD raised significantly after 14 days. Simultaneously did the level of P2H, P2M, P5 and the control drop.

The ALP levels of the peptides followed a similar trend to the control group, although here did the P2 groups show the highest levels of the groups, meanwhile EMD had a significantly lower level. Also for this gene expression did P2L have the improved performance. This along with the ALP activity test suggests that the synthetic peptides have better cell proliferation than the amelogenin derived EDM.

A common bone formation marker is osteocalcin, released by the osteoblast cells during osteoid synthesis; however, it is also reported to rapidly degrade both *in vivo* and *in vitro*. [J.P. Brown, C. Albert, B.A. Nassar, J.D. Adachi, D. Cole, K.S. Davison, K.C. Dooley, A. Don-Wauchope, P. Douville, D.A. Hanley, S.A. Jamal, R. Josse, S. Kaiser, J. Krahn, R. Krause, R. Kremer, R. Lepage, E. Letendre, S. Morin, D.S. Ooi, A. Papaioaonnou, L.-G. Ste-Marie, Bone turnover markers in the management of postmenopausal osteoporosis, Clinical Biochemistry 42(10) (2009) 929-942.]. For P2H, P2M, P6 and EMD is the OC level the highest after 7 days before it drops for the second week. This suggest a high early osteoblast activity for these groups. For P2L and control the max is reached after 14 days, meanwhile P5 is fairly stable.

In terms of VEGFA did the peptides initially have a better performance than the control group, however after 7 and 14 days did the control group show better levels than the remaining groups. Although P2L followed some of the same trend as the control-group.

**Example 6. In vivo experiments**

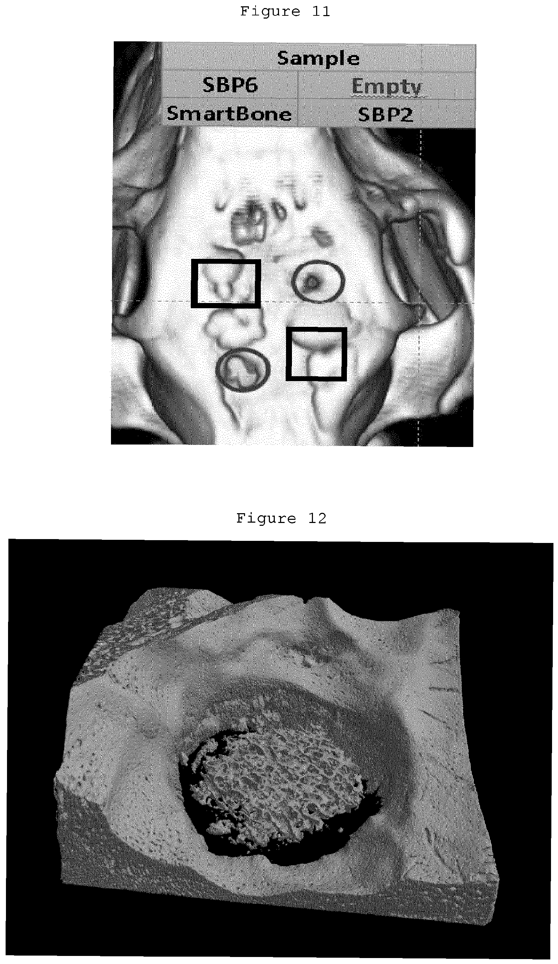

Animal experiment was conducted with 18 hybrid pig, Age: 3-4 months and quarantine for 28 days prior to surgery. All the experiments was be carried out in accordance with the national legislation following the community guidelines after the authorization of the competent autonomous authority in the facilities available to the Rof Codina Foundation for this purpose. All procedures was be performed using general anaesthesia according to standard protocols. The material for testing is the as stated in example 2a. A longitudinal incision was made at the level of the frontal bones and the muscles was reflected. Then, 4 perforations of 2 mm in diameter was made at the level of the bone plate under continuous irrigation with sterile saline. Said perforations was sealed with the use of different tests or controls and will close muscular, subcutaneous and skin plane, allowing to heal for 4 weeks. Four defects with 16 mm in diameter was made with a specialized burr. The positive control was Smartbone®, a bone implant matrix, reproducing the technology according to the PCT application no. PCT/IB2009/007759, corresponding to the comparative embodiment of Example 3 of the present application, and negative control empty space sham. The defects, except sham, had a bovine pericardium membrane (Tutopatch, Tissue Matrix, Tutogen Medical GmbH) except sham. Defects were created in the middle line at the level of the frontal and parietal bones and was filled with the material to be tested. Antibiotic prophylaxis was administered for one week using amoxicillin (20 mg / kg / s.i.d. / s.c.). There was two timepoint 8 week and 16 weeks. 9 animals was sacrificed by an overdose of pentobarbital (40-60 mg / kg / i.v.) after previous sedation after 8 weeks and 9 animals after 16 weeks. The skulls was dissected and the bone blocks with the defects under study was obtained, fixing them in a 10% buffered formaldehyde solution for 4-7 days at 4oC. A cone beam and microtomographic study was performed of the defects and their percentage of regeneration was carried out. The results (Figure 11: the CBCT images of SBP variants with SEQ ID 4 (SBP2) and SEQ ID 9 (SBP6) show enhanced bone growth around the defects with the peptide sequences 4 and 9 (SBP6:, black box right , SBP2: black box left, empty (negative control): circle right and Smartbone® (positive control): circle left, respectively and Figure 12: a skull defect with SBP variants with SEQ ID 4 (SBP2) showing ingrowth of bone into the defect) showed enhanced bone formation and faster closure of the defects when both compared to negative and positive controls. All defects with the peptide sequences had significantly thicker and more bone formation in the defects when compared to sham.

**Discussion**

It has already been established that the bone implant matrix, identified as "SB", according to the PCT application no. PCT/IB2009/007759, herewith reproduced according to the example 3 had a microstructure similar to healthy iliac bones with an average of 27% porosity, and that that the rigged structure of the said "SB" ensures better osteointegration than just a bovine based xenograft [G. Pertici, F. Rossi, T. Casalini, G. Perale, Composite polymer-coated mineral grafts for bone regeneration: material characterisation and model study, Annals of Oral & Maxillofacial Surgery 2(1) (2014)]. By analysing the reinforcing mixture coating of the bone implant matrix "SBP" according to the present invention (such as those according to examples 2A - 2E), it has been confirmed that the microstructure is equivalent to that of the above cited "SB" and there was a homogenous distribution of the peptide in the reinforcing mixture coating. This is a fairly basic coating structure, as natural tissue doesn't have a homogenous structure [J. Leijten, Y.C. Chai, I. Papantoniou, L. Geris, J. Schrooten, F. Luyten, Cell based advanced therapeutic medicinal products for bone repair: keep it simple?, Adv Drug Deliver Rev 84 (2015) 30-44.]. Ideally, growth factors should promote tissue growth and mineralization such that the regenerated tissue has similar structure to the native tissue environment. This may be difficult with homogenous distribution of conventional growth factors such as BMP or EDM, however "PRP" has been designed to have an automatic biological on-and-off function. This allows the "PRP" to be active only when required.

Bone require its composite cells to be at different stages of proliferation, differentiation and maturation in a multilayered organised structure to promote successful tissue growth [D. Tang, R.S. Tare, L.Y. Yang, D.F. Williams, K.L. Ou, R.O.C. Oreffo, Biofabrication of bone tissue: approaches, challenges and translation for bone regeneration, Biomaterials 83 (2016) 363-382.]. Therefore, it is important to tune the peptide release rate such that there is a high early growth, but also reassure stage diversity for the cells. The target release for the peptide availability was $1\mu$g (per cc "SBP") per day for one week, as experimentally proven. This should give a release of $10\mu$g in 14 days with a peak in the first 2 days. As seen in the results, both Seq. ID 4 and Seq. ID 8 had its highest release rate the two first days and after 14 days was the release of Seq. ID 4 around $11.75$-$14.01\mu$g and $8.75$-$9.90\mu$g for Seq. ID 8. This confirms that the release was as designed. Hence the bone implant matrix "SBP" according to the present invention (such as those according to examples 2A - 2E), has a peptide release rate which is very adequate to paediatric bone regeneration.

Considering the mRNA level for the "PRP" and ALP activity it is strong indications of that the peptide presents higher osteoblast differentiation and promotes osteogenesis. Moreover, the significantly higher ALP activity for "PRP" than the amelogenin derived EMD illustrates the enormous potential of IDP when it comes to the clinical success of tissue regeneration. Particularly poor osseointegration and tissue formation has of current available graft is driving the need for enhanced growth and improved yield of newly formed bone [D. Tang, R.S. Tare, L.Y. Yang, D.F. Williams, K.L. Ou, R.O.C. Oreffo, Biofabrication of bone tissue: approaches, challenges and translation for bone regeneration, Biomaterials 83 (2016) 363-382]. Therefore, the presence of "PRP" in the reinforcing mixture coating of the bone implant matrix "SBP" according to the present invention (such as those according to examples 2A - 2E) is a good step in the right direction.

**Claims**

1. A bone implant matrix comprising:

a base matrix the surface thereof is coated with a statistically homogeneous composition which is a reinforcing mixture containing at least a soluble polymer, at least a substance, which is able to promote the cell-rooting and the cell growth, by stimulating cell proliferation and tissue integration ("friendliness to cell") and at least one artificial Proline-Rich Peptide wherein the base matrix is selected from the group comprising:

- acellularized or acellularized non-demineralised bone matrix of any source,
- matrix of natural mineral sources,
- synthetic bioceramics matrix,

or mixtures thereof,
the soluble polymer of the reinforcing mixture is a biodegradable polyester or co-polymer thereof, the "friendliness to cell" is selected from the group consisting of gelatine, hydrolysed gelatine and the artificial Proline-Rich Peptide is selected from: an artificial peptide comprising the amino acid sequence of Pro-X-X-Pro-Y-Y-Y-Pro-X-X-Pro-Y-Y-Pro-X-X-Pro-X-Pro-Y-Y-Y-Y-Y-Y-Pro-Y-Y-Y-Y-Y-Pro-X-X-Pro-X-Pro-Y-Y-Y-Pro-Y-Y-Pro-Y-Pro-X-X-Pro -Y-Pro- Y-Y-Pro-X-X-Pro-Y-Y-Pro-X-X-Pro-Y-Y -Pro-X-X -Pro-Y-Pro-Pro-X-Pro-Pro -X-X-X-X-X-X-X-X-Pro-X-X-Pro-X-X-X-X (SEQ ID NO 1), wherein:

a) Pro is proline;
b) X is an amino acid independently selected from the group consisting of Ala, Ile, Leu, Met, Phe, Trp and Val, preferably Ile, Leu, Val and Met;
c) Y, is an amino acid independently selected from the group consisting of Asn, Cys, Gln, Ser, Thr and Tyr, preferably Ser and Gln.

or
an artificial peptide comprising the amino acid sequence of Pro-X-X-Pro-Y-Y-Pro-X-X-Pro-Y-Y- Pro-X-X-Pro-Y-Y-Pro-Y-Pro-Pro-X-Pro-Pro (SEQ ID NO 2), wherein:

a) Pro is proline;
b) X is an amino acid independently selected from the group consisting of Ala, Ile, Leu, Met, Phe, Trp and Val;
c) Y is an amino acid independently selected from the group consisting of Asn, Cys, Gln, Ser, Thr and Tyr.

2. A bone implant matrix according to claim 1, wherein the bio-degradable polyester or copolymer thereof is selected from the group consisting of polylactic acid (PLA), polyglycolic acid (PGA), polycaprolactone (PCL) and co-polymers thereof comprising polycaprolactone-polylactic (PLA/PCL) co-polymers, poly(L-lactide-co- -caprolactone) co-polymers, poly(L-lactide), poly(D,L-lactide-co-glycolide), poly(L-lactide-co-D,L-lactide), their enantiomers, their co-polymers and mixtures thereof.

3. A bone implant matrix according to claim 1, wherein the biodegradable polyester or co-polymer thereof is selected from the group consisting of poly(caprolactones), poly(L-lactide), poly(D,L-lactide-co-glycolide), poly(L-lactide-co-D,L-lactide), their enantiomers, their co-polymers and mixtures thereof.

4. A bone implant matrix according to claim 1, wherein the biodegradable polyester or co-polymer thereof is selected from the group consisting of polycaprolactone-polylactic copolymer (PLA/PCL) or poly(L-lactide-co-ε-caprolactone) co-polymer.

5. A bone implant matrix according to claim 1, wherein the artificial Proline-Rich Peptide is an artificial peptide comprising the amino acid sequence selected from the group comprising:

PLV PSY PLV PSY PLV PSY PYP PLPP (SEQ ID NO 3),
PLV PSQ PLV PSQ PLV PSQ PQP PLPP (SEQ ID NO 4),
PLV PCC PLV PCC PLV PCC PCP PLPP (SEQ ID NO 5),
PMM PSY PMM PSY PMM PSY PYP PMPP (SEQ ID NO 6),
PLV PSS PLV PSS PLV PSS PSP PLPP (SEQ ID NO 7),
PLV PSS PLV PCC PLV PCC PSP PLPP (SEQ ID NO 8),
PHQ PMQ PQP PVH PMQ PLP PQP PLPP (SEQ ID NO 9)

or a combination thereof.

6. A bone implant matrix according to claim 1, wherein the artificial Proline-Rich Peptide is an artificial peptide comprising the amino acid sequence selected from the group comprising:

PLV PSQ PLV PSQ PLV PSQ PQP PLPP (SEQ ID NO 4),
PLV PSS PLV PCC PLV PCC PSP PLPP (SEQ ID NO 8),
PHQ PMQ PQP PVH PMQ PLP PQP PLPP (SEQ ID NO 9)

or a combination of the amino acid sequence of:

PLVP SQ PLV PSQ PLV PSQ PQP PLPP (SEQ ID NO 4) and
PLV PSS PLV PCC PLV PCC PSP PLPP (SEQ ID NO 8),
PLV PSQ PLV PSQ PLV PSQ PQP PLPP (SEQ ID NO 4) and
PHQ PMQ PQP PVH PMQ PLP PQP PLPP (SEQ ID NO 9),
PLV PSS PLV PCC PLV PCC PSP PLPP (SEQ ID NO 8) and
PHQ PMQ PQP PVH PMQ PLP PQP PLPP (SEQ ID NO 9).

7. A bone implant matrix according to claim 1, wherein the artificial Proline-Rich Peptide is an artificial peptide comprising the amino acid sequence of:
PHQ PMQ PQP PVH PMQ PLP PQP PLPP (SEQ ID NO 9).

8. A bone implant matrix according to claim 1, wherein the artificial Proline-Rich Peptide is an artificial peptide comprising the amino acid sequence of:
PLV PSQ PLV PSQ PLV PSQ PQP PLPP (SEQ ID NO 4).

9. A bone implant matrix according to claim 1, wherein the artificial Proline-Rich Peptide is a combination of the amino acid sequence of:

PLV PSQ PLV PSQ PLV PSQ PQP PLPP (SEQ ID NO 4) and
PHQ PMQ PQP PVH PMQ PLP PQP PLPP (SEQ ID NO 9).

10. A bone implant matrix according to claim 1, wherein the acellularized or acellularized non-demineralised bone matrix of any source is elected from the group comprising:

- acellularized or acellularized non-demineralised human-derived bone matrix,
- acellularized or acellularized non-demineralised xeno-derived bone matrix, such as acellularized or acellularized non-demineralised bovine-bone matrix, acellularized or acellularized non-demineralised equine-bone matrix, acellularized or acellularized non-demineralised porcine-bone matrix.

11. A bone implant matrix according to claim 1, wherein the matrix of natural mineral sources is selected from the group comprising mother of pearl, coral, nacre.

12. A bone implant matrix according to claim 1, wherein the synthetic bioceramics matrix is selected from the group comprising hydroxyl-apatites, calcium carbonates, calcium phosphates, silicon oxides, titanium oxides, aluminium oxides, zirconia oxides, graphites, bioglasses.

13. A bone implant matrix according to claim 1, comprising an acellularised bovine bone matrix, coated with a reinforcing mixture comprising a biodegradable polyester-based copolymer, hydrolysed gelatine/gelatine and an artificial Proline-Rich Peptide which is an artificial peptide comprising the amino acid sequence selected from the group comprising:

PLV PSY PLV PSY PLV PSY PYP PLPP (SEQ ID NO 3),
PLV PSQ PLV PSQ PLV PSQ PQP PLPP (SEQ ID NO 4),
PLV PCC PLV PCC PLV PCC PCP PLPP (SEQ ID NO 5),
PMM PSY PMM PSY PMM PSY PYP PMPP (SEQ ID NO 6),
PLV PSS PLV PSS PLV PSS PSP PLPP (SEQ ID NO 7),
PLV PSS PLV PCC PLV PCC PSP PLPP (SEQ ID NO 8),
PHQ PMQ PQP PVH PMQ PLP PQP PLPP (SEQ ID NO 9)

or a combination thereof.

**14.** A bone implant matrix according to claim 1, comprising an acellularised non-demineralised bovine bone matrix, coated with a reinforcing mixture comprising a biodegradable polyester-based copolymer, hydrolysed gelatine/gelatine and an artificial Proline-Rich Peptide which is an artificial peptide comprising the amino acid sequence selected form the group comprising:

> PLV PSY PLV PSY PLV PSY PYP PLPP (SEQ ID NO 3),
> PLV PSQ PLV PSQ PLV PSQ PQP PLPP (SEQ ID NO 4),
> PLV PCC PLV PCC PLV PCC PCP PLPP (SEQ ID NO 5),
> PMM PSY PMM PSY PMM PSY PYP PMPP (SEQ ID NO 6),
> PLV PSS PLV PSS PLV PSS PSP PLPP (SEQ ID NO 7),
> PLV PSS PLV PCC PLV PCC PSP PLPP (SEQ ID NO 8),
> PHQ PMQ PQP PVH PMQ PLP PQP PLPP (SEQ ID NO 9)

or a combination thereof.

**15.** A bone implant matrix according to claims 13-14, wherein the biodegradable polyester-based co-polymer is a polycaprolactone-polylactic copolymer (PLA/PCL).

**16.** A bone implant matrix according to claims 13-14, wherein the biodegradable polyester-based co-polymer is a poly(L-lactide-co- -caprolacton e) co-polymer.

**17.** A bone implant matrix according to claims 13-14, wherein the artificial Proline-Rich Peptide is an artificial peptide comprising the amino acid sequence selected from the group comprising:

> PLV PSQ PLV PSQ PLV PSQ PQP PLPP (SEQ ID NO 4),
> PLV PSS PLV PCC PLV PCC PSP PLPP (SEQ ID NO 8),
> PHQ PMQ PQP PVH PMQ PLP PQP PLPP (SEQ ID NO 9)

or a combination of the amino acid sequence of:

> PLV PSQ PLV PSQ PLV PSQ PQP PLPP (SEQ ID NO 4) and
> PLV PSS PLV PCC PLV PCC PSP PLPP (SEQ ID NO 8),
> PLV PSQ PLV PSQ PLV PSQ PQP PLPP (SEQ ID NO 4) and
> PHQ PMQ PQP PVH PMQ PLP PQP PLPP (SEQ ID NO 9),
> PLV PSS PLV PCC PLV PCC PSP PLPP (SEQ ID NO 8) and
> PHQ PMQ PQP PVH PMQ PLP PQP PLPP (SEQ ID NO 9).

**18.** A bone implant matrix according to claims 13-14, wherein the artificial Proline-Rich Peptide is an artificial peptide comprising the amino acid sequence of:
PHQ PMQ PQP PVH PMQ PLP PQP PLPP (SEQ ID NO 9).

**19.** A bone implant matrix according to claim 1, for use in at least one of the following fields: bone reconstructive surgery, in bone regeneration surgery, in regenerative surgery, in skull and maxillo-facial bone reconstructive surgery, in oncologic surgery, pediatric cases, in oral surgery, dental surgery, orthopaedic surgery, spine surgery, traumatology and implantology.

**20.** A bone implant matrix according to claim 1, for use in human or veterinary treatment.

Figure 1

Figure 2

Figure 3

Figure 4

Figure 5

Figure 6

Figure 7

Figure 8

Figure 9

Figure 10

Figure 11

Figure 12

Europäisches
Patentamt

European
Patent Office

Office européen
des brevets

# EUROPEAN SEARCH REPORT

Application Number

EP 19 16 5892

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/070416 A1 (IND BIOMEDICHE INSUBRI S A [CH]; PERTICI GIANNI [CH]) 24 June 2010 (2010-06-24) * page 12, line 24 - page 13, line 12 * * examples 2A, 2B, 2C * * claims * ----- | 1-20 | INV. A61L27/10 A61L27/12 A61L27/34 A61L27/36 A61L27/42 A61L27/54 A61L27/58 |
| A | WO 2014/044704 A1 (CORTICALIS AS [NO]) 27 March 2014 (2014-03-27) * page 12, line 14 - page 13, line 31 * * page 23, lines 3-7 * * page 24, line 20 - page 25, line 11 * * examples 1-8 * * claims * ----- | 1-20 | |

TECHNICAL FIELDS
SEARCHED (IPC)

A61L

The present search report has been drawn up for all claims

| Place of search | Date of completion of the search | Examiner |
|---|---|---|
| The Hague | 25 September 2019 | Van den Bulcke, H |

EPO FORM 1503 03.82 (P04C01)

## ANNEX TO THE EUROPEAN SEARCH REPORT
## ON EUROPEAN PATENT APPLICATION NO.

EP 19 16 5892

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2019

| Patent document cited in search report | | Publication date | Patent family member(s) | Publication date |
|---|---|---|---|---|
| WO 2010070416 | A1 | 24-06-2010 | BR PI0923017 A2 | 15-12-2015 |
| | | | CH 700138 A1 | 30-06-2010 |
| | | | CN 102256633 A | 23-11-2011 |
| | | | CN 106890361 A | 27-06-2017 |
| | | | EP 2358407 A1 | 24-08-2011 |
| | | | ES 2557105 T3 | 22-01-2016 |
| | | | JP 5801719 B2 | 28-10-2015 |
| | | | JP 2012512684 A | 07-06-2012 |
| | | | RU 2011126095 A | 27-01-2013 |
| | | | US 2011218646 A1 | 08-09-2011 |
| | | | WO 2010070416 A1 | 24-06-2010 |
| | | | ZA 201103322 B | 25-07-2012 |
| WO 2014044704 | A1 | 27-03-2014 | CA 2880998 A1 | 27-03-2014 |
| | | | EP 2897656 A1 | 29-07-2015 |
| | | | ES 2608777 T3 | 17-04-2017 |
| | | | JP 6169178 B2 | 26-07-2017 |
| | | | JP 2015529527 A | 08-10-2015 |
| | | | KR 20150058202 A | 28-05-2015 |
| | | | PL 2897656 T3 | 31-03-2017 |
| | | | SE 1251043 A1 | 19-03-2014 |
| | | | US 2015250923 A1 | 10-09-2015 |
| | | | WO 2014044704 A1 | 27-03-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO IB2009007759 A

**Non-patent literature cited in the description**

- **Y. FILLINGHAM ; J. JACOBS.** Bone grafts and their substitutes. *The bone & joint journal,* 2016, vol. 98 (1), 6-9
- **L. ROSETI ; V. PARISI ; M. PETRETTA ; C. CAVALLO ; G. DESANDO ; I. BARTOLOTTI ; B. GRIGOLO.** Scaffolds for Bone Tissue Engineering: State of the art and new perspectives. *Mater Sci Eng C,* 2017, vol. 78, 1246-1262
- **R. AGARWAL ; A.J. GARCIA.** Biomaterial strategies for engineering implants for enhanced osseointegration and bone repair. *Adv Drug Deliver Rev,* 2015, vol. 94, 53-62
- **D.L. SKAGGS ; M.A. SAMUELSON ; J.M. HALE ; R.M. KAY ; V.T. TOLO.** Complications of posterior iliac crest bone grafting in spine surgery in children. *Spine,* 2000, vol. 25 (18), 2400-2402
- **B. HUANG ; G. CAETANO ; C. VYAS ; J.J. BLAKER ; C. DIVER ; P. BÁRTOLO.** Polymer-Ceramic Composite Scaffolds: The Effect of Hydroxyapatite and -tri-Calcium Phosphate. *Materials,* 2018, vol. 11 (1), 129
- **M. DOMINGOS ; A. GLORIA ; J. COELHO ; P. BARTOLO ; J. CIURANA.** Three-dimensional printed bone scaffolds: The role of nano/micro-hydroxyapatite particles on the adhesion and differentiation of human mesenchymal stem cells. *Proceedings of the Institution of Mechanical Engineers, Part H: Journal of Engineering in Medicine,* 2017, vol. 231 (6), 555-564
- **G. HANNINK ; J.C. ARTS.** Bioresorbability, porosity and mechanical strength of bone substitutes: what is optimal for bone regeneration?. *Injury,* 2011, vol. 42, S22-S25
- **S. CORBELLA ; S. TASCHIERI ; R. WEINSTEIN ; M. DEL FABBRO.** Histomorphometric outcomes after lateral sinus floor elevation procedure: a systematic review of the literature and meta-analysis. *Clin Oral Implan Res,* 2016, vol. 27 (9), 1106-1122

- **L. CORDARO ; D.D. BOSSHARDT ; P. PALATTELLA ; W. RAO ; G. SERINO ; M. CHIA-PASCO.** Maxillary sinus grafting with Bio-Oss® or Straumann® Bone Ceramic: histomorphometric results from a randomized controlled multicenter clinical trial. *Clin Oral Implan Res,* 2008, vol. 19 (8), 796-803
- **A. ALDAADAA ; N. OWJI ; J. KNOWLES.** Three-dimensional Printing in Maxillofacial Surgery: Hype versus Reality. *Journal of tissue engineering,* 2018, vol. 9
- **P.-E. FOURNIER ; R. RIZZOLI ; D.O. SLOSMAN ; G. THEINTZ ; J.-P. BONJOUR.** Asynchrony between the rates of standing height gain and bone mass accumulation during puberty. *Osteoporosis Int,* 1997, vol. 7 (6), 525-532
- **N.E. PICARDO ; G.W. BLUNN ; A.S. SHEKKERIS ; J. MESWANIA ; W.J. ASTON ; R.C. POLLOCK ; J.A. SKINNER ; S.R. CANNON ; T.W. BRIGGS.** The medium-term results of the Stanmore non-invasive extendible endoprosthesis in the treatment of paediatric bone tumours. *The Journal of Bone and Joint Surgery,* 2012, vol. 94-B (3), 425-430
- **P.-E. FOURNIER ; R. RIZZOLI ; D.O. SLOSMAN ; G. THEINTZ ; J.P. BONJOUR.** Asynchrony between the rates of standing height gain and bone mass accumulation during puberty. *Osteoporosis Int,* 1997, vol. 7 (6), 525-532
- **K.M. EMARA ; R.A. DIAB ; A.K. EMARA.** Recent biological trends in management of fracture non-union. *World journal of orthopedics,* 2015, vol. 6 (8), 623
- **S. BORAIAH ; O. PAUL ; D. HAWKES ; M. WICKHAM ; D.G. LORICH.** Complications of recombinant human BMP-2 for treating complex tibial plateau fractures: a preliminary report. *Clinical Orthopaedics and Related Research®,* 2009, vol. 467 (12), 3257-3262
- **L. KALMAR ; D. HOMOLA ; G. VARGA ; P. TOMPA.** Structural disorder in proteins brings order to crystal growth in biomineralization. *Bone,* 2012, vol. 51 (3), 528-534

- **M. WOJTAS ; P. DOBRYSZYCKI ; A. OŻYHAR.** Intrinsically disordered proteins in biomineralization, Advanced Topics in Biomineralization. *InTech,* 2012
- **M. WOJTAS ; P. DOBRYSZYCKI ; A. O Y HAR.** Intrinsically disordered proteins in biomineralization, Advanced Topics in Biomineralization. *InTech,* 2012
- **J. HABCHI ; P. TOMPA ; S. LONGHI ; V.N. UVERSKY.** Introducing protein intrinsic disorder. *Chemical reviews,* 2014, vol. 114 (13), 6561-6588
- **ELSHARKAWY ; M. AL-JAWAD ; M.F. PANTANO ; E. TEJEDA-MONTES ; K. MEHTA ; H. JAMAL ; S. AGARWAL ; K. SHUTURMINSKA ; A. RICE ; N.V. TARAKINA.** Protein disorder-order interplay to guide the growth of hierarchical mineralized structures. *Nature communications,* 2018, vol. 9 (1), 2145
- **G. PERALE ; P. AROSIO ; D. MOSCATELLI ; V. BARRI ; M. MÜLLER ; S. MACCAGNAN ; M. MASI.** A new model of resorbable device degradation and drug release: transient 1-dimension diffusional model. *Journal of Controlled Release,* 2009, vol. 136 (3), 196-205
- **M. GOMEZ-FLORIT ; M. RUBERT ; J.M. RAMIS ; H.J. HAUGEN ; H. TIAINEN ; S.P. LYNGSTADAAS ; M. MONJO.** TiO2 Scaffolds Sustain Differentiation of MC3T3-E1 Cells. *J Biomater Tiss Eng,* 2012, vol. 2 (4), 336-344
- **G. PERTICI ; F. ROSSI ; T. CASALINI ; G. PERALE.** Composite polymer-coated mineral grafts for bone regeneration: material characterisation and model study. *Annals of Oral & Maxillofacial Surgery,* 2014, vol. 2 (1
- **K. CHATZIPANAGIS ; M. LAFISCO ; T. RONCAL-HERRERO ; M. BILTON ; A. TAMPIERI ; R. KRÖGER ; J.M. DELGADO-LOPEZ.** Crystallization of citrate-stabilized amorphous calcium phosphate to nanocrystalline apatite: a surface-mediated transformation. *CrystEngComm,* 2016, vol. 18 (18), 3170-3173
- **J.P. BROWN ; C. ALBERT ; B.A. NASSAR ; J.D. ADACHI ; D. COLE ; K.S. DAVISON ; K.C. DOOLEY ; A. DON-WAUCHOPE ; P. DOUVILLE ; D.A. HANLEY.** Bone turnover markers in the management of postmenopausal osteoporosis. *Clinical Biochemistry,* 2009, vol. 42 (10), 929-942
- **J. LEIJTEN ; Y.C. CHAI ; I. PAPANTONIOU ; L. GERIS ; J. SCHROOTEN ; F. LUYTEN.** Cell based advanced therapeutic medicinal products for bone repair: keep it simple?. *Adv Drug Deliver Rev,* 2015, vol. 84, 30-44
- **D. TANG ; R.S. TARE ; L.Y. YANG ; D.F. WILLIAMS ; K.L. OU ; R.O.C. OREFFO.** Biofabrication of bone tissue: approaches, challenges and translation for bone regeneration. *Biomaterials,* 2016, vol. 83, 363-382